# EUROPEAN PATENT SPECIFICATION

(11) **EP 3 366 303 B1**
(45) Date of publication and mention of the grant of the patent: **24.06.2020**
(21) Application number: 17158463.4
(22) Date of filing: 28.02.2017
(51) Int. Cl.: A61K 38/18, A61K 31/09, A61K 31/337, A61K 31/513, A61K 31/675, A61K 31/704, A61K 31/7068, A61K 35/17, A61K 35/28, A61P 35/00, A61P 25/00, A61P 29/00

(54) **ENHANCING THE TARGETED IN VIVO DELIVERY OF CELLULAR THERAPIES**
VERBESSERUNG DER GEZIELTEN IN VIVO VERABRECHUNG VON ZELLULAREN THERAPIEN
AMÉLIORER LA LIVRAISON CIBLÉE IN VIVO DE THERAPIES CELLULAIRES

(43) Date of publication of application: 29.08.2018
(73) Proprietor: Martin-Luther-Universität Halle-Wittenberg, 06108 Halle/Saale (DE)
(72) Inventor: HERMANN, Felix, 81375 München (DE); GÜNTHER, Christine, 81479 München (DE); MÜLLER, Thomas, 06108 Halle (DE)
(74) Representative: Hertin und Partner Rechts- und Patentanwälte PartG mbB

(56) References cited:
- WO-A1-2008/150368
- WO-A1-2016/065330
- L. BRACCI ET AL: "Cyclophosphamide Enhances the Antitumor Efficacy of Adoptively Transferred Immune Cells through the Induction of Cytokine Expression, B-Cell and T-Cell Homeostatic Proliferation, and Specific Tumor Infiltration", CLINICAL CANCER RESEARCH, vol. 13, no. 2, 15 January 2007 (2007-01-15), pages 644-653, XP055380616, US ISSN: 1078-0432, DOI: 10.1158/1078-0432.CCR-06-1209
- L G M DAENEN ET AL: "Treatment-induced host-mediated mechanisms reducing the efficacy of antitumor therapies", ONCOGENE, vol. 33, no. 11, 25 March 2013 (2013-03-25), pages 1341-1347, XP055380610, ISSN: 0950-9232, DOI: 10.1038/onc.2013.94
- YUVAL SHAKED ET AL: "Rapid Chemotherapy-Induced Acute Endothelial Progenitor Cell Mobilization: Implications for Antiangiogenic Drugs as Chemosensitizing Agents", CANCER CELL, vol. 14, no. 3, 1 September 2008 (2008-09-01), pages 263-273, XP055380601, US ISSN: 1535-6108, DOI: 10.1016/j.ccr.2008.08.001
- LOTTE B BERTELSEN ET AL: "Treatment with a vascular disrupting agent does not increase recruitment of indium labelled human endothelial outgrowth cells in an experimental tumour model", BMC CANCER, BIOMED CENTRAL, LONDON, GB, vol. 14, no. 1, 2 December 2014 (2014-12-02), page 903, XP021204731, ISSN: 1471-2407, DOI: 10.1186/1471-2407-14-903
- CARMEN S M YONG ET AL: "CAR T-cell therapy of solid tumors", IMMUNOLOGY AND CELL BIOLOGY, vol. 95, no. 4, 22 December 2016 (2016-12-22), pages 356-363, XP055380406, AU ISSN: 0818-9641, DOI: 10.1038/icb.2016.128
- ANN DE BECKER ET AL: "Homing and migration of mesenchymal stromal cells: How to improve the efficacy of cell therapy?", WORLD JOURNAL OF STEM CELLS, vol. 8, no. 3, 1 January 2016 (2016-01-01) , page 73, XP055380458, CN ISSN: 1948-0210, DOI: 10.4252/wjsc.v8.i3.73
- SHAKED Y ET AL: "Therapy-induced acute recruitment of circlating endothelial progenitor cells to tumors", SCIENCE, AMERICAN ASSOCIATION FOR THE ADVANCEMENT OF SCIENCE, vol. 313, no. 5794, 22 September 2006 (2006-09-22), pages 1785-1787, XP002501939, ISSN: 0036-8075, DOI: 10.1126/SCIENCE.1127592

## Description

The invention relates to the technical field of cellular therapy, preferably mesenchymal stem cell (MSC) or T cell therapy, and their use in the treatment of medical conditions such as tumors diseases or inflammation. The invention relates to a vascular endothelial damaging agent for use as an adjuvant to increase the therapeutic effect of a cellular therapy in the treatment of a medical condition. Preferred agents that damage and/or are toxic to the endothelium are cytostatic agents, immunosuppressant agents, more preferably Combretastatins or analogues thereof.

In one embodiment the invention relates to treating, preferably pretreating, tumor patients with agents that damage and/or are toxic to the endothelium in the tumor vasculature, in order to enhance the tumor-specific enrichment of mesenchymal stem cells (MSCs). In another embodiment the invention relates to treating, preferably pretreating, patients suffering from a medical condition associated with inflammation with agents that damage and/or are toxic to the vascular endothelium, in order to enhance the enrichment of MSCs at one or more sites of inflammation.

The invention therefore relates to a cell therapy product comprising mammalian therapeutic cells, preferably mesenchymal stem cell (MSCs) or T cells, for use as a medicament in the treatment of a medical condition, for the treatment of a tumor and/or malignant disease or a disease associated with inflammation, wherein said treatment comprises administration of a vascular endothelial damaging agent to the subject prior to or in combination with the cell therapy product. In one embodiment the prior or combined administration of a vascular endothelial damaging agent leads to enrichment of therapeutic cells in vivo at sites of interest, such as tumors or sites of inflammation.

### BACKGROUND OF THE INVENTION

Mesenchymal stem cells (MSCs) are cells of non-haematopoietic origin that reside in the bone marrow and other tissues. The correct identification and nomenclature of the MSC has been clarified by the Mesenchymal and Tissue Stem Cell Committee of the International Society for Cellular Therapy (ISCT), who have proposed a set of minimal criteria to define MSC: Firstly, MSC have to grow in a plastic-adherent manner when maintained under standard cell culture conditions. Secondly, the cells have to express a panel of the following surface markers: CD105, CD73, and CD90 expression (≥95%). On the other hand a set of specific marker must be absent: CD45, CD34, CD14, or CD11b, CD79alpha or CD19 and HLA-DR (≤2% positive). Furthermore, the MSCs must be able to differentiate into osteoblasts, adipocytes, and chondroblasts when cultured under standard in vitro differentiating conditions [M. Dominici, K. Le Blanc, I. Mueller et al., "Minimal criteria for defining multipotent mesenchymal stromal cells. The International Society for Cellular Therapy position statement," Cytotherapy, vol. 8, no. 4, pp. 315-317, 2006.].

MSC have been shown to selectively migrate to tumor sites or sites of injury and inflammation after intravenous application in various animal models (Review: Leibacher and Henschler, Biodistribution, migration and homing of systemically applied mesenchymal stem/ stromal cells Stem Cell Research & Therapy (2016) 7:7). The cells are therefore suitable to deliver therapeutic active gene / proteins to sites of inflammation or tumors. This concept has been shown to work efficiently in various preclinical tumor models (Sage, K.E. et al., Cytotherapy. 2016; 18(11): 1435-1445). MSC have been used in preclinical models to transfer therapeutic genes to tumors and elicit an anti-tumor effect. They have further been successfully used in animal models of inflammation-associated autoimmune diseases and graft-vs-host disease. The efficacy in these models may also be attributed at least in part to their ability to home to sites of inflammation after systemic application (Klinker et al., World J Stem Cells. (2015) 7(3): 556-567, Choi (2009) Int J Stem Cells. 2(2): 122-128).

The successful application of MSC for the treatment of tumor indication and inflammatory diseases has been hampered by the low level of specific enrichment of MSC at the target site (tumor or site of inflammation). It has been estimated that a small fraction of the applied MSC dose reaches the tumor site (Karp et al., Cell Stem Cell 4, 2009). The low frequency of tumor homing and homing to sites of inflammation reduces the potential therapeutic effect.

Similar obstacle exist for T cell-based therapy approaches (adoptive T cell transfers and CAR-T cells) to treat tumors. Artificial T cell receptors (also known as chimeric T cell receptors, chimeric immunereceptors, chimeric antigen receptors (CARs)) are engineered receptors, which graft an arbitrary specificity onto an immune cell. Typically, these receptors are used to graft the specificity of a monoclonal antibody onto a T cell and to activate that T cell upon cognate antigen engagement; with transfer of their coding sequence facilitated by retro- or lentiviral vectors.

Artificial T cell receptors are under investigation as a therapy for cancer, using a technique called adoptive cell transfer. T cells are removed from a patient and modified ex vivo so that they express receptors specific to the particular form of cancer. The T cells, which can then recognize and kill the cancer cells, are administered into the patient with or without pre-conditioning of the patient. Allogeneic T cells engineered in the same way and sourced from donors other than the patient are also under investigation.

The successful application of CAR-T cells to treat solid tumors has proven very difficult, although these cells are very promising for the treatment of soluble tumors such as leukemias. One of the reasons for the lack of efficacy of CAR-T cells for the application of solid tumors is the low percentage of T cells which reach the tumors. For CAR-T cells (reviewed by Fesnak et al. (2016) Nature Reviews Cancer 16, 566-581) it can be estimated that likely less than 1% of the applied CAR-T cells is able to infiltrate the target solid tumor in animal models. Rabinovic et al (Rabinovic et al. (2008) PNAS, 105, pp. 14342-14346) have shown that only approx. 0.5% of the applied T cell dose can be found in a murine tumor model. Similar conclusions can be drawn from the data published by Curuana et al (Curuana et al. (2015) Nature Medicine 21, 524-529). The authors have used a murine tumor model and have shown that only approximately 0.5% of the applied CAR-T cells dose can be found in a tumor 3-5 days after systemic T cell application.

One of the major hurdles which hamper efficient tumor-specific recruitment or recruitment to the sites of injury after systemic application of cell-based therapies is the endothelium. Sackstein et al. have shown that the surface receptors on MSC under normal conditions do not allow efficient binding to endothelial cells. This binding is a prerequisite to allow extravasation. Sackstein at al. have modified the surface of MSC to enhance their ability to bind to endothelial cells. The modification leads to improved binding of MSC to endothelial ligand E-selectin and allows recruitment of MSC to the vessels in the bone marrow with improved efficacy (Sackstein et al., Nature Medicine 14, 181 - 187 (2008)).

Various chemotherapeutic agents which are clinically applied to treat tumors are toxic to the endothelium. They are currently considered as potential angiogenesis inhibitors, although they were not originally developed as such, representing the so-called "accidental" anti-angiogenic drugs. (Review: A. Soultati et al./Cancer Treatment Reviews 38 (2012) 473-483, Endothelial vascular toxicity from chemotherapeutic agents: Preclinical evidence and clinical implications). In addition, combretastatin analogues have been identified to have endothelium damaging properties (WO 2011/138409).

The tumour vasculature is an attractive target for therapy. Combretastatin A-4 (CA-4) and A-1 (CA-1) are tubulin binding agents, structurally related to colchicine, which induce vascular-mediated tumour necrosis in animal models. CA-1 and CA-4 were isolated from the African bush willow. More soluble, phosphated, forms of CA-4 (CA-4-P) and CA-1 (CA-1-P) are commonly used for in vitro and in vivo studies. These are cleaved to the natural forms by endogenous phosphatases and are taken up into cells (Tozer et al., Int J Exp Pathol. 2002 Feb; 83(1): 21-38).

In addition, therapeutic modalities which are used for the treatment of inflammatory diseases (i.e. immunosupressants) are also known to have endothelium damaging side effects. For example, Cyclosporine A leads to endothelial phenotypic changes and death (Bouvier et al., (2009), Am J Physiol Renal Physiol 296: F160-F169) and Leflunomide is an apporoved immunosupresant which is known to be toxic to the endothelium. (Waldman et al. (2001), Transplantation. 15;72(9):1578-82, and Zeng L et al., (2008), Transplant Proc., 40(8):2670-3).

In light of promising cell therapy approaches towards treatment of inflammation and cancer using MSC or T cell-based therapy and the difficulties in achieving enrichment of these therapeutic cellular agents at the site of a tumor or inflammation, a strong need exists in the field of cellular therapeutics for enhancing the enrichment of cellular agents at sites *in vivo* where their therapeutic effect is required.

### SUMMARY OF THE INVENTION

In light of the prior art, the technical problem underlying the present invention is to improve the enrichment of a therapeutic cell product at a relevant disease site in vivo, such as at a tumor or the site of inflammation. A further problem underlying the invention is the provision of means for enhancing the activity of MSC or T cell based therapies. A further problem underlying the invention is the provision of means for enhancing CAR-T activity.

The problem of the invention is solved by the subject matter of the independent claims. Preferred embodiments of the invention are represented by the dependent claims.

The invention therefore relates to a cell therapy product comprising mammalian therapeutic cells for use in the treatment of a solid tumor disease, a disease associated with inflammation or a disorder of the central nervous system (CNS) or neurological or neurodegenerative disorder, wherein the use of said cell therapy product comprises administration of a vascular endothelial damaging agent prior to or in combination with administration of said cell therapy product.

It was entirely surprising that a cell therapy product comprising mammalian therapeutic cells, preferably mesenchymal stem cell (MSCs) or T cells, can be enhanced by the prior or combined use of an endothelial damaging agent. The endothelial damage appears to lead to an enrichment of therapeutic cells at the relevant site in vivo.

For example, regions of inflammation or tumors may be preferentially targeted by the endothelium damaging agents. A skilled person would not have expected from the prior art or his knowledge in the field that a "preparation" of the endothelium in a tumor or site of inflammation, by causing some degree of damage of the endothelium, would lead to enhanced function, preferably via enhanced enrichment of therapeutic cells, for example after systemic administration.

In some embodiments the endothelium damaging agent is determined using the assays for assessing endothelium damage as described herein.

In some embodiments of the invention the endothelium damage induced by said endothelium damaging agent is such that the endothelium is structurally and/or functionally affected by the treatment, but not to an extent that leads to general discomfort or illness of the subject of treatment, or preferably does not lead to such damage as to be classified as a medical condition.

In some embodiments of the invention the endothelial damaging agent leads to a localized damage of the endothelium in tumors and/or at sites of inflammation. The concentration of the endothelial damaging agent can be adjusted in order to meet these needs. For example, the vasculature of tumors typically is not optimally formed when compared to healthy vasculature, for example due to fast tumor growth and deregulation of normal endothelial vasculature development in order to enable tumor growth. The endothelium of the tumor may therefore be particularly susceptible to endothelial damage by the agents described herein, and as such a tumor-specific damage can be achieved by adjusting the concentration of the agent appropriately. Furthermore, the vasculature endothelium of an area of inflammation may also be specifically targeted by the agents described herein, as the blood flow to areas of inflammation is increased in comparison to normal tissue. Due to this innate physiological effect, the inflamed areas of the body may be "specifically" damaged or damaged "to an enhanced extent" compared to non-inflamed tissues.

In one embodiment of the invention the endothelial damaging agent is administered as an adjuvant in an amount and/or in a manner effective to enhance the therapeutic effect of said therapeutic cells.

In one embodiment of the invention the endothelial damaging agent is administered as an adjuvant in an amount and/or in a manner effective to enhance the enrichment of said therapeutic cells at a target site in the subject of treatment.

In one embodiment of the invention the endothelial damaging agent is a cytostatic agent.

In one embodiment of the invention the endothelial damaging agent is a combretastatin or an analogue or derivative thereof.

In one embodiment of the invention the combretastatin or an analogue or derivative thereof is 1-Methyl-5-(3-amino-4-ethoxyphenyl)-4-(3-bromo-4,5-dimethoxyphenyl)-imidazol bis(hydrochlorid), otherwise named "Ethyl-Brimamin".

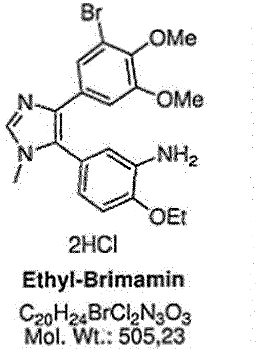

In one embodiment of the invention the endothelial damaging agent is a compound of a general structural formula or specific compound as disclosed in WO2011/138409.

In one embodiment of the invention the endothelial damaging agent is selected from the group consisting of platinum compounds, cyclophosphamide, anthracyclines, gemcitabine, Doxorubicin, Daunorubicin, bleomycin, 5-fluorouracil, taxanes or methotrexate and/or analogues thereof that exhibit the endothelium damaging effect.

In one embodiment of the invention the endothelial damaging agent is an immunosuppressant. In one embodiment of the invention the cell therapy product is administered from 0.5 to 72 hours, preferably 1 to 24 hours, more preferably 2 to 12 hours after administration of said endothelial damaging agent.

According to the invention the cell therapy product is administered in the treatment of a solid tumor disease.

According to the invention the cell therapy product is administered in the treatment of a disease associated with inflammation.

In one embodiment of the invention the cell therapy product comprises or consists of mesenchymal stem cells (MSCs).

In one embodiment of the invention the MSCs are genetically modified to express a therapeutic gene product from an exogenous nucleic acid.

In one embodiment of the invention the cellular therapy product comprises or consists of T cells.

In one embodiment of the invention the T cells are chimeric antigen receptor T cells (CAR-Ts).

In one embodiment of the invention the cell therapy product is administered systemically, preferably intravenously, intra-arterially and/or intraperitoneally.

In one embodiment of the invention the MSCs and CAR-Ts are administered in combination in one or more cell therapy products.

In a further aspect, the invention therefore also relates to an endothelial damaging agent for use as an adjuvant to enhancing the therapeutic effect of a cellular therapy in the treatment of a medical condition, preferably to enhance the enrichment of said therapeutic cells at a target site in the subject of treatment. The features of the embodiments described above are therefore also encompassed by the invention in the context of an endothelial damaging agent for use as an adjuvant in a medical treatment.

The invention therefore also relates to a method for enhancing the therapeutic effect of a cellular therapy in the treatment of a medical condition, preferably enhancing the enrichment of said therapeutic cells at a target site in the subject of treatment, wherein said cellular therapy comprises the administration of therapeutic cells in the treatment of medical condition, preferably selected from cancer, such as a solid tumor, or a medical condition associated with inflammation.

### Enhancement of Drug delivery across the Blood-Brain-Barrier

The blood-brain barrier (BBB) poses a unique challenge for drug delivery to the central nervous system (CNS). The BBB consists of a continuous layer of specialized endothelial cells linked together by tight junctions. This complex barrier controls and limits the systemic delivery of therapeutics to the CNS. The BBB serves an important role by maintaining homeostasis and preventing macromolecules, infectious agents, and potential neurotoxins from entering the brain. However, more than 90% of all small-molecule drugs and nearly 100% of all larger therapeutics are prevented from crossing the BBB. New means are therefore needed for enhancing BBB permeability to therapeutic agents.

Also disclosed herein is the use of an endothelial damaging agent as described herein for enhancing the permeability of the blood-brain-barrier to active agents. Also disclosed herein is that the endothelial damaging agent may be administered in advance or in combination with any given therapeutic agent. According to the invention the therapeutic agent is a therapeutic cell, such as those cellular therapies described herein, preferably an MSC or immune cell.

As is reviewed in Hersh et al. (Curr Pharm Des. 2016; 22(9): 1177-1193), cellular therapy is a viable option for enabling therapeutic delivery across the BBB. Immune cells and stem cells have been identified as potential agents that cross the BBB that can act as therapeutic agents themselves or deliver therapeutic proteins or other agents. Both immune cells and stem cells, such as MSCs, have been demonstrated to cross the BBB and migrate into the CNS via an inflammation-mediated pathway. Cells can deliver a variety of therapeutics, including genes, cytokines, enzymes, and nanoparticles. Moreover, immune cells and stem cells are naturally recruited to sites of tissue damage and inflammation, a common feature of diseases of the CNS, including brain cancer of inflammatory diseases of the CNS. Intravenous administration of these cell types has resulted in accumulation in brain tumors. Immune cells are known to migrate across endothelial barriers, including the BBB, by a process known as diapedesis, whereby the cell transiently tethers to and rolls along the endothelial cells and ultimately transmigrates through interactions between integrins, cell adhesion molecules and selectin molecules. MSCs express many of the same types of receptors and adhesion molecules as immune cells and may enter in a similar manner, although unlike immune cells, MSCs do not move laterally along the endothelial wall, and MSCs cross endothelial barriers much more slowly. Despite cellular therapies, as agents themselves or as vehicles, being able to cross the BBB, enhancement of their progress into the CNS in the treatment of diseases of the CNS or neurological diseases, are required.

According to the present invention endothelial damaging agents may be employed to enhance the permeability of the BBB to therapeutic agents, such as therapeutic cells. As is described above, a number of substances, such as cytostatic or immunosuppressant agents, have been shown to induce some extent of endothelial damage after systemic administration. This damage can be used to enrich cellular therapies at the site of tumors or inflammation, but can also be harnessed to permeabilize the BBB to other active compounds. In one embodiment, the combretastatins and analogues thereof as described herein, such as those described in WO 2011/138409, lead to a mild toxic effect on the endothelium, capable of enabling stem or immune cell enrichment and ultimately passage of therapeutic agents, such as stem or immune cells, through the endothelium of the BBB.

Also disclosed herein is an endothelial damaging agent for use as an adjuvant to increase the therapeutic effect of a therapeutic agent in the treatment of a medical condition, in which the crossing of the BBB and/or enrichment of therapeutic agent in the CNS or brain is advantageous during therapy. Preferred agents that damage and/or are toxic to the endothelium are cytostatic agents, immunosuppressant agents, more preferably Combretastatins or analogues thereof, as described herein.

In one embodiment the invention relates to treating, preferably pretreating, patients with CNS or neurological disorders, or any other disorder in which enhanced delivery of a therapeutic agent across the BBB would be advantageous, with agents that damage and/or are toxic to the endothelium. In one embodiment the subject may have a tumor of the brain. In one embodiment the therapeutic agent may be MSCs. In another embodiment the invention relates to treating, preferably pretreating, patients suffering from a medical condition associated with inflammation in the CNS or a neurodegenerative disease with agents that damage and/or are toxic to the endothelium, in order to enhance the enrichment of a therapeutic agent, preferably a cellular agent such as an immune cell or MSCs, at one or more sites of disease in the brain or in the CNS.

The invention therefore relates to a cell therapy product comprising mammalian therapeutic cells, preferably mesenchymal stem cell (MSCs) or T cells, for use as a medicament in the treatment of a medical condition, for the treatment of a tumor and/or malignant disease or a disease associated with inflammation in the brain or CNS, wherein said treatment comprises administration of an endothelial damaging agent to the subject prior to or in combination with the cell therapy product. In one embodiment the prior or combined administration of an endothelial damaging agent leads to enrichment of therapeutic cells in vivo at sites of interest and/or the enhanced permeability of the BBB.

The invention further relates to a composition comprising a cell therapy product comprising mammalian therapeutic cells and a vascular endothelial damaging agent for use as a medicament.

The invention further relates to a combination of a cell therapy product comprising mammalian therapeutic cells and a vascular endothelial damaging agent, wherein said cell therapy product and vascular endothelial damaging agent are packaged together for immediate combined use as a medicament.

### DETAILED DESCRIPTION OF THE INVENTION

The invention is characterised by the surprising finding that a cell therapy product comprising mammalian therapeutic cells, preferably mesenchymal stem cell (MSCs) or T cells, can be enhanced by the prior or combined use of an endothelial damaging agent. The endothelial damage appears to lead to an enrichment of therapeutic cells at the relevant site in vivo. For example, regions of inflammation or tumors may be preferentially targeted by the endothelium damaging agents. The tumor vasculature is particularly susceptible to endothelial damage. The damaged endothelium thereby acts as a target site for enhanced enrichment, engraftment, homing and/or localization of the therapeutic cells to the region of interest. A skilled person could not have derived from the prior art that the prior or combined administration of an endothelial damaging agent would lead to enrichment of therapeutic cells in vivo at sites of interest, such as tumors or sites of inflammation.

### Cell therapy product:

A cell therapy product relates in one embodiment to a pharmaceutical product or composition that comprises one or more therapeutic cells. Cellular therapy has been established as a promising approach towards the treatment of various medical disorders. For example, stem cells or immune cells may be administered as or in a cell therapy product to a patient to alleviate illness. Mammalian therapeutic cells are obtained from a mammalian subject, preferably a human, according to techniques known to a skilled person in the art.

Adult stem cells are routinely used in medical therapies, for example in bone marrow transplantation. Medical researchers anticipate that various types of stem cells are able to treat cancer, Type 1 diabetes mellitus, Parkinson's disease, Huntington's disease, Celiac disease, cardiac failure, muscle damage and neurological disorders, and many others. Adult stem cells may be lineage-restricted (multipotent) and are generally referred to by their tissue origin (mesenchymal stem cell, adipose-derived stem cell, endothelial stem cell, etc.).

### Endothelial damaging agent:

According to the present invention an endothelial damaging agent is a chemical compound, a substance, either naturally occurring or synthetic, or composition comprising such a compound or substance, that exhibits a toxic or damaging effect to the endothelium of a subject, preferably to the endothelium of the vasculature. In particular, the endothelial damaging agent exhibits a toxic effect on the endothelium of the vascular system of a tumor and/or the endothelium of the vascular system at a site of or in proximity to inflammation.

Examples of compounds with endothelium damaging properties are Combretastatins, Combretastatin analogues or derivatives, platinum compounds, cyclophosphamide, anthracyclines, gemcitabine, Doxorubicin, Daunorubicin, bleomycin, 5-fluorouracil, taxanes or methotrexate and/or derivatives or analogues thereof that exhibit the endothelium damaging effect.

The endothelium represents an epithelium that lines the interior surface of blood vessels and lymphatic vessels in mammals, thereby forming a boundary and interface between circulating blood or lymph in the lumen and the rest of the vessel wall. It is a thin layer of cells called endothelial cells. Vascular endothelial cells line the circulatory system, from the heart to the smallest capillaries.

The inventors have found that an endothelium damaging agent leads to "preparation" of the endothelium, for example the vascular endothelium in a tumor, or the endothelium at a site of inflammation, which enable therapeutic cells such as stem cells or immune cells, e. g. MSCs or T cells, to more effectively enrich in the endothelium, for example in the tumor tissue or stroma. After enrichment and/or engraftment of the therapeutic cells their therapeutic effect can be realized more effectively in a local manner, thereby enhancing the local and site specific therapeutic action of the cells.

The desired effect of an endothelial damaging agent can be determined by a skilled person, for example by assessing enrichment of therapeutic cells administered in a subject, or in an animal model. Suitable techniques for assessing cell engraftment are disclosed below.

Furthermore, the endothelial damaging effect of the present invention may be tested for example by employing a HUVEC network assay, an MTT-Assay with HUVEC or subcutaneous and orthotopic tumor models, or an in vivo model to measure endothelial damage, namely the model may be performed as described by Guba et al. (Nat Med. 2002, 8(2):128-35). Through using such assays and/or models an endothelial damaging agent may be identified by its desired function.

### In vitro assays for determining the endothelium damaging effect of any given agent:

### HUVEC network assay:

Human umbilical vein endothelial cell (HUVEC) are obtained from Sigma Aldrich or can be isolated according to Jaffe et al (Jaffe E, Nachman R, Becker C and Minick C (1973) Culture of human endothelial cells derived from umbilical veins. J Clin Invest 52 : 2745-2756). Isolated HUVEC are grown in medium M199 (Sigma Aldrich) supplemented with 10% human serum and 100 mM sodium pyruvate, 200 mM L-glutamine, 5000 IU/ml penicillin and 5000 µg/ml streptomycin. All culture flasks used are coated with 0.2% gelatin in phosphate-buffered saline (PBS).

Type I calf-skin collagen solution (900 µl of 1.1 mg/ml in 0.01 N acetic acid) is mixed with 100 µl of 10 x (ten times concentrated) MEM and 150 µl of freshly prepared 7.5% (w/v) sodium bicarbonate solution. This collagen solution (300 µl) is then added to each well, in 24-well plates and incubated at 37 ° C until the gels set (approximately 15-20 min). Pre-warmed culture medium is then added to wells and the gels incubated for 3 h to equilibrate. After the 3-h incubation period the medium is removed and replaced with 0.5 ml of fresh pre-warmed medium, containing 1 x 10⁵ HUVEC cells ml -1 and the gels are incubated for a further 24 h (37 ° C, 5% carbon dioxide). The medium is then carefully removed and a second layer of collagen, of the same composition as the first one, was added and left to set before addition of fresh pre-warmed medium. The following day HUVEC networks could be observed (K Grosios et al., British Journal of Cancer (1999) 81 (8), 1318-1327).

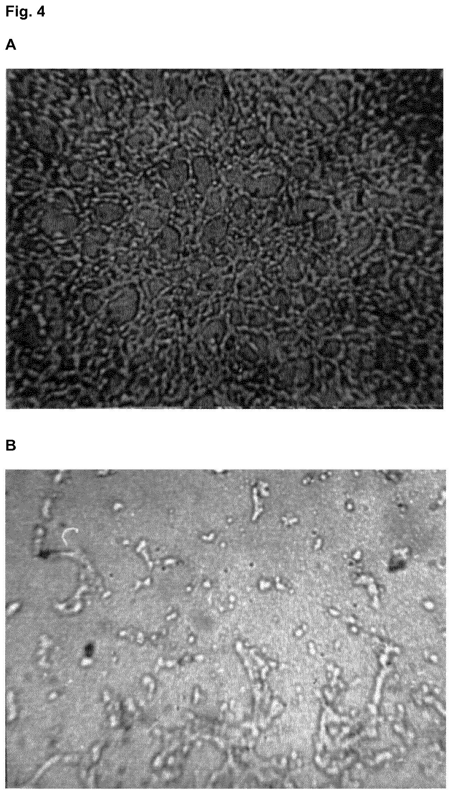

To test substances for their capacity to damage endothelium, substances are dissolved in water or DMSO and are added to the established HUVEC networks. The samples are incubated at 37 ° C, 5% carbon dioxide. If the substance in the tested concentration is damaging to the endothelial cells, the network structure break down occurs in the next 4 to 24h. The breakdown of the networks is analyzed microscopically (refer Figure 4).

### MTT-Assay with HUVEC:

Mitochondrial activity of living HUVECs as sign for viability, can be measured by performing a 3-(4,5-dimethylthiazol- 2-yl)2,5- diphenyl-tetrazolium bromide (MTT) assay (SigmaAldrich Co., St. Louis, MO, USA) as described by Annussek et al. (Annussek, Szuwart, Kleinheinz, Koiky and Wermker (2014) In vitro inhibition of HUVECs by low dose methotrexate - insights into oral adverse events. Head & Face Medicine 2014, 10:19). HUVEC are seeded into 24-well plates (between 200 - 1000 cells per well) and are incubated for 24h at 37 ° C, 5% carbon dioxide.

Afterwards, the medium is exchanged for medium containing the compound to be tested in various concentrations (0nM - 5000nM). The cells are incubated for 24h at 37 ° C, 5% carbon dioxide. Afterwards, cells are incubated for one hour with 1 ml (MTT) solution (0.25 mg/ml medium) at 37°C in a humidified atmosphere with 5% CO2. Within incubation time living cells metabolize the MTT to formazan crystals. Thereafter, medium is aspirated and 200 µl of propanol is added to lyse the cells and dissolve the released formazan crystals. To complete dissolution of the formazan salts, plates are placed on a vibrating platform shaker for 5 - 30 min. The extinction value is measured at a wavelength of 570 nm using an enzyme-linked immunosorbent assay reader (µQuant, Biotek instruments, Bad Friedrichshall, Germany or similar).

### In vivo model to measure endothelial damage:

The model may be performed as described by Guba et al. (Guba M, von Breitenbuch P, Steinbauer M, Koehl G, Flegel S, Hornung M, Bruns CJ, Zuelke C, Farkas S, Anthuber M, Jauch KW, Geissler EK.(2002) Rapamycin inhibits primary and metastatic tumor growth by antiangiogenesis: involvement of vascular endothelial growth factor. Nat Med. 8(2):128-35).

Subcutaneous and orthotopic tumor models. To establish tumors in mice, 10⁶ tumor cells are injected subcutaneously in the mid-dorsal region of immunodeficient mice (e.g. nude mice). Tumors were allowed to grow for 7 days without treatment and then treatment with an endothelial damaging agent is initiated. Tumor volumes are estimated by measurements of the short and long tumor axis with a caliper. Mice are sacrificed on day 12; tumors are then removed, measured and prepared for immunohistology.

Immunofluorescent staining of blood vessels. Cryosections of tumors are stained for endothelial cells with a rat monoclonal antibody specific for mouse CD31 (Pharmingen, San Diego, CA) 38 . The secondary antibody is a goat immunoglobulin specific for rat, conjugated to Texas Red (Jackson ImmunoResearch, West Grove, PA). CD31-staining vessels are quantified in 10 random microscopic fields per tumor by an independent observer. CD31 signal is the marker for presence of blood vessles. Reduced signal in treated animals compared to untreated animals indicats endothelial damage.

### Administration of endothelium damaging agent:

The endothelium damaging agent may preferably be administered prior to the cell therapy product. Administration "prior to" refers to any administration of an endothelium damaging agent in advance of the cellular therapy. For example, the endothelium damaging agent may be administered from 1 minute to 1 month prior to the cell therapy, from 5 minutes to 1 week prior to the cellular therapy, or from 0.5 to 72 hours, preferably 1 to 24 hours, more preferably 2 to 12 hours prior to administration of the therapeutic cells.

The endothelium damaging agent may be administered in combination with the cell therapy. "Combined administration" may relate to concurrent and/or sequential administration of said therapeutic cells prior to, during and/or subsequent to said endothelium damaging agent. Combined treatment shall also include a combination treatment regime comprising multiple administrations of either component of the treatment. Combined administration encompasses simultaneous treatment, co-treatment or joint treatment, for example when the two components are prepared in a single composition or are packaged together for immediate combined use, and also includes the administration of separate formulations of therapeutic cells with an endothelium damaging agent, whereby treatment may occur within minutes of each other, in the same hour, on the same day, in the same week or in the same month as one another. Sequential administration of any given combination of combined agents is also encompassed by the term "combined administration".

An adjuvant relates typically to a pharmacological or immunological agent that modifies the effect of other agents. Adjuvants may typically be added to a vaccine to boost the immune response. In the present invention the adjuvant (endothelium damaging agent) leads preferably to an enhanced effect of the cellular therapy that is measureable compared to when no adjuvant is applied.

The endothelium damaging agent is preferably administered in an amount and/or in a manner effective to enhance the therapeutic effect of said therapeutic cells. In one embodiment this is an amount and/or in a manner effective to enhance the enrichment, engraftment, homing or localization of said therapeutic cells at a target site in the subject of treatment. A skilled person is capable of determining the necessary doses of said agent and measuring the therapeutic effect of the agent by standard means. Therapeutic effect can be determined by appropriate in vivo or in vitro methods and will depend on the disease to be treated and therapeutic cell employed.

In one embodiment the endothelium damaging agent is a cytostatic agent. Various cytostatic agents are known to a skilled person capable of causing endothelium damage, such as, but not limited to, those provided herein.

### Combretastatins and analogues or derivatives thereof:

In one embodiment of the invention the endothelial damaging agent is a combretastatin or an analogue or derivative thereof. Combretastatins are a class of natural phenols. A variety of different natural combretastatin molecules are present in the bark of Combretum caffrum, commonly known as South African Bush Willow. Combretastatins generally share 3 common structural features: a trimethoxy "A"-ring, a "B"-ring containing substitutents often at C3' and C4', and an ethene bridge between the two rings. Alternative bridge structures have been designed and also prove effective. Examples of Combretastatins are Combretastatin A-1, Combretastatin B-1 and Combretastatin A-4.

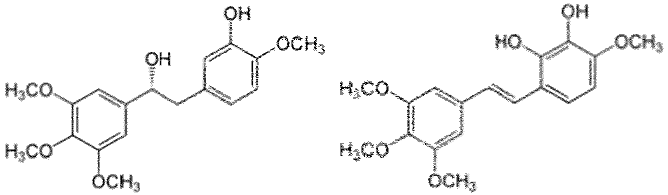

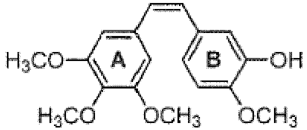

According to one embodiment of the present invention, the combretastatin or an analogue or derivative thereof relates to a compound described in WO2011/138409. The relevant WO publication discloses methods for synthesis of the relevant compounds and preferred substances.

The combretastatin or an analogue or derivative thereof preferably relates to a compound of formula (I) 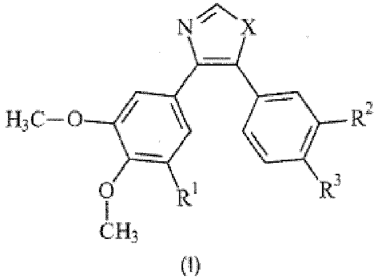 or a pharmaceutically acceptable salt, solvate, or prodrug thereof.
X is selected from O, S, N(H), or N(C1-4 alkyl). Preferably, X is selected from O or N(C1-4 alkyl). More preferably, X is selected from O or N(CH3).
R1 is selected from halogen, -CN, -CF3, -NH2, -NH(C1-4 alkyl), or -N(C1-4 alkyl)(C1-4 alkyl). Preferably, R1 is selected from halogen (such as, e.g., -F, -CI, or -Br), or -NH2. More preferably, R1 is selected from -CI, -Br, or -NH2. Most preferably, R1 is selected from -CI or -Br.
R2 is selected from hydrogen, halogen, -CN, -CF3, -OH, -O(C1-4 alkyl), -NH2, -NH(C1-4 alkyl), or -N(C1-4 alkyl)(C1-4 alkyl). Preferably, R2 is selected from hydrogen, halogen (such as, e.g., -F, - CI, or-Br), -OH, or -NH2. More preferably, R2 is selected from -F, -OH, or -NH2.
R3 is selected from -OH, -O(C1-4 alkyl), -SH, -S(C1-4 alkyl), -NH2, -NH(C1-4 alkyl), or-N(C1-4alkyl)(C1-4 alkyl). Preferably, R3 is selected from -O(C1-4 alkyl) or -N(C1-4 alkyl)(C1-4 alkyl). More preferably, R3 is selected from -O-CH3, -O-CH2-CH3 or -N(CH3)2, particularly from -O-CH3 or -N(CH3)2.

Alternatively, R2 and R3 jointly form a group -C(halogen)=CH-N(CH3)-, wherein the halogen is preferably selected from -F, -CI, or -Br, and more preferably the halogen is -CI. That is, R2 and R3 form a 5-membered ring together with the carbon atoms which they are attached to, wherein R2 and R3 together are a bivalent group -C(halogen)=CH-N(CH3)- or, preferably, a group -C(CI)=CH-N(CH3)-.

Accordingly, the compound of formula (I) may have the following structure: 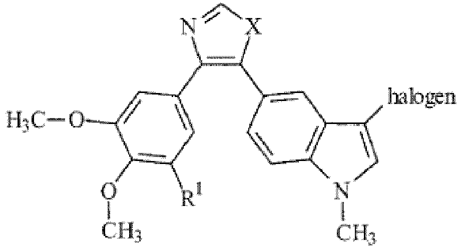 wherein the halogen may, e.g., be -F, -CI, or -Br, and preferably is -CI, and the other groups X and R1 are as defined above.

In a preferred embodiment, R2 is -NH2 and R3 is -O-CH3. In a further preferred embodiment, X is O or N(CH3), R1 is -NH2 or halogen (such as, e.g., -F, -CI, or -Br), R2 is -NH2, and R3 is -O-CH3. Accordingly, a preferred compound of formula (I) is 1-methyl-5-(3-amino-4-methoxyphenyl)-4-(3-chloro-4,5-dimethoxyphenyl)-imidazole (also referred to as "5b" or "compound 5b"). Another preferred compound of formula (I) is 1 -methyl-5-(3-amino-4-methoxyphenyl)-4-(3-bromo-4,5-dimethoxyphenyl)-imidazole (also referred to as "6b" or "compound 6b").

In another preferred embodiment, R2 is -NH2 and R3 is -O-CH2-CH3. In a further preferred embodiment, X is O or N(CH3), R1 is -NH2 or halogen (such as, e.g., -F, -CI, or -Br), R2 is -NH2, and R3 is -O-CH2-CH3. Particularly preferred compounds of formula (I) are, accordingly, 1-methyl-5-(3-amino-4-ethoxyphenyi)-4-(3-chloro-4,5-dimethoxyphenyl)-imidazole (i.e., 1-methyl-5-(3"-amino-4"-ethoxyphenyl)-4-(3'-chloro-4',5'-dimethoxyphenyl)-imidazole; also referred to as "5f" or "compound 5f") and 1-methyl-5-(3-amino-4-ethoxyphenyl)-4-(3-bromo-4,5-dimethoxyphenyl)-imidazole (i.e., 1-methyl-5-(3"-amino-4"-ethoxyphenyi)-4-(3'-bromo-4',5'-dimethoxyphenyl)-imidazole; also referred to as "6F" or "compound 6f").

In another preferred embodiment, R2 is -F and R3 is -O-CH3 or -O-CH2-CH3. In a. further preferred embodiment, X is O or N(CH3), R1 is -NH2 or halogen (such as, e.g., -Cl, or -Br), R2 is -F, and R3 is -O-CH3 or -O-CH2-CH3. Accordingly, preferred compounds of formula (I) are 1-methyl-4-(3-chloro-4,5-dimethoxyphenyl)-5-(3-fluoro-4-ethoxyphenyl)-imidazole (also referred to as "5g" or "compound 5g") and 1-methyl-4-(3-bromo-4,5-dimethoxyphenyl)-5-(3-fiuoro-4-ethoxyphenyl)-imidazole (also referred to as "6g" or "compound 6g").

Further preferred compounds of formula (I) are 1-methyl-4-(3-amino-4,5-dimethoxyphenyi)-5- (N-methyl-3-chloroindol-5-yl)-imidazole (i.e., 1-methyl-4-(3'-amino-4',5'-dimethoxyphenyl)-5- (N-methyl-3"-chloroindol-5"-yl)-imidazole; also referred to as "8e" or "compound 8e"), 1-methyl-4-(3'-chioro-4',5'-dimethoxyphenyl)-5-(N-methyl-3"-chloroindol-5"-yl)-imidazole (also referred to as "5i" or "compound 5i"), and 1-methyl-4-(3'-bromo-4',5'-dimethoxyphenyl)-5-(N- methyl-3"-chloroindol-5"-yl)-imidazole (also referred to as "6i" or "compound 6i").

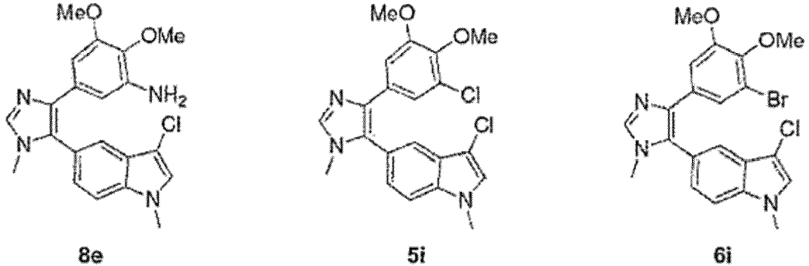

The combretastatin or an analogue or derivative thereof preferably relates to a compound of formula (II) 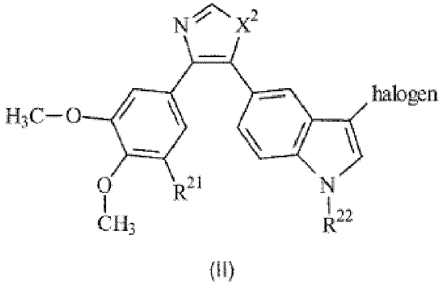 or a pharmaceutically acceptable salt, solvate, or prodrug thereof.
X2 Is selected from O, S, N(H), or N(C1-4 alkyl). Preferably, X2 is selected from O or N(C1-4 alkyl). More preferably, X2 is selected from O or N(CH3).
R21 is selected from halogen, -CN, -CF3, -NH2, -NH(C1-4 alkyl), or -N(C1-4 alkyl)(C1-4 alkyl). Preferably, R21 is selected from halogen (such as, e.g., -F, -CI, or -Br), or -NH2. More preferably, R21 is selected from -CI, -Br, or -NH2. Most preferably, R21 is selected from -CI or-Br.
R22 is C1-4 alkyl. Preferably, R22 is C2-4 alkyl. More preferably, R22 is ethyl.

The group "halogen" comprised in the compound of formula (II) is preferably selected from -F, - CI, or-Br, and more preferably the "halogen" is -CI.

Preferred compounds of formula (II) are 1 -methyl-4-(3-amino-4,5-dimethoxyphenyl)-5-(N-methyl-3-chloroindol-5-yl)-imidazole, 1-methyl-4-(3'-chloro-4',5'-dimethoxyphenyl)-5-(N-methyl-3"-chloroindol-5"-yl)-imidazole, and 1-methyl-4-(3'-bromo-4',5'-dimethoxyphenyl)-5-(N-methyl-3"-chloroindol-5"-yl)-imidazole. A particularly preferred compound of formula (II) is 1-methyl-4-(3'-bromo-4',5'-dimethoxyphenyl)-5-(N-ethyi-3"-chloroindol-5"-yl)-imidazole (also referred to as "6h" or "compound 6h").

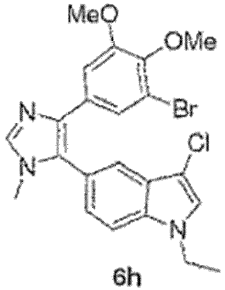

The combretastatin or an analogue or derivative thereof preferably relates to a compound of formula (III) 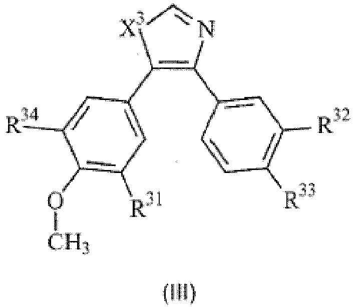 or a pharmaceutically acceptable salt, solvate, or prodrug thereof.
X3 is selected from O, S, N(H), or (C1-4 alkyl). Preferably, X3 is selected from O or N(C1-4 alkyl). More preferably, X3 is selected from O or N(CH3).
R31 is selected from halogen (particularly -Cl, -Br or -I), -CN, -CF3, -NH2, - NH(C1-4 alkyl), or-N(C1-4 alkyl)(C1-4 alkyl). Preferably, R31 is selected from -CI, -Br, -I, or -NH2. More preferably, R31 is selected from -Br, -I, or -NH2. Most preferably, R31 is selected from -Br or -I.
R32 is selected from hydrogen, halogen, -CN, -CF3, -OH, -O(C1-4 alkyl), -NH2, -NH(C1-4 alkyl), or-N(C1-4 alkyl)(C1-4 alkyl). Preferably, R32 is selected from hydrogen, halogen (such as, e.g., - F, -CI, or -Br), -OH, or -NH2. More preferably, R32 is selected from -F, -OH, or -NH2. Most preferably, R32 is -F.
R33 is selected from -OH, -O(C1-4 alkyl), -SH, -S(C1-4 alkyl), -NH2, -NH(C1-4 alkyl), or-N(C1-4 alkyl)(C1-4 alkyl). Preferably, R33 is selected from -O(C1-4 alkyl) or -N(C1-4 alkyl)(C1-4 alkyl).

More preferably, R33 is selected from -O-CH3 O-CH2-CH3 or -N(CH3)2. Most preferably, R33 is selected from -O-CH3 or -O-CH2-CH3.

Alternatively, R32 and R33 jointly form a group -C(halogen)=CH-N(C1-4 alkyl)-. The C1-4 alkyl comprised in that group is preferably selected from methyl or ethyl. The halogen comprised in that group is preferably selected from -F, -CI, or -Br, and more preferably the halogen is -CI. That is, R32 and R33 form a 5-membered ring together with the carbon atoms which they are attached to, wherein R32 and R33 together are a bivalent group -C(halogen)=CH-N(C1-4 alkyl)-. It is particularly preferred that R32 and R33 jointly form a group -C(halogen)=CH-N(CH3)- or a group - C(halogen)=CH-N(CH2CH3)-, wherein in each case the halogen is preferably selected from -F, - CI, or-Br, and more preferably the halogen is -CI.

R34 is selected from -OH, -O(C1-4 alkyl), -SH, -S(C1-4 alkyl), halogen (particularly -CI, -Br or -I), -CN, -CF3, -NH2, -NH(C1-4 alkyl), or -N(C1-4 alkyl)(C1-4 alkyl). Preferably, R34 is selected from -O(C1-4 alkyl), -CI, -Br, -I, or -NH2. More preferably, R34 is selected from -O-CH3, -O-CH2-CH3, -Br, -I, or -NH2. Most preferably, R34 is selected from -O-CH3, -Br or -I.

In a preferred embodiment, R is -F and R is -O-CH3. In a further preferred embodiment, X3 is N(CH3), R31 is -Br or -I, R32 is -F, R33 is -O-CH3, and R34 is -O-CH3, -Br or -I. Particularly preferred compounds of formula (III) are the following compounds 9a, 9b and 9c:

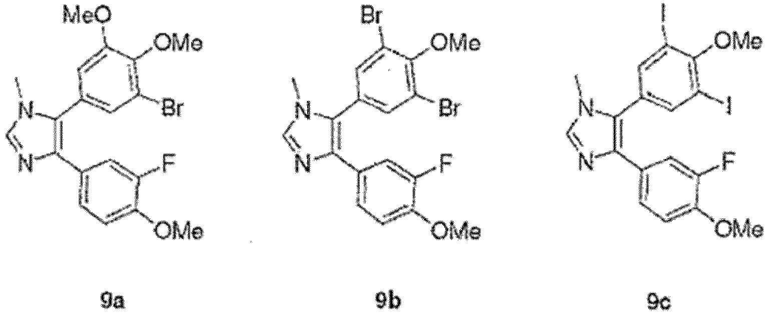

As used herein, the term "alkyl" refers to a monovalent saturated aliphatic (i.e. non-aromatic) acyclic hydrocarbon group (i.e. a group consisting of carbon atoms and hydrogen atoms) which may be linear or branched and does not comprise any carbon-to-carbon double bond or any carbon-to-carbon triple bond. Accordingly, the term "C1-4 alkyl" refers to methyl, ethyl, propyl (e.g., n-propyl or isopropyl), or butyl (e.g., n-butyl, isobutyl, tert-butyl, or sec-butyl).

As used herein, the term "halogen" refers to -F, -CI, -Br, or -I, and in particular to -F, -CI, or -Br.

The present invention also relates to a pharmaceutical composition for use according to the present invention comprising a compound of formula (I), (II) or (III) as defined herein or a pharmaceutically acceptable salt, solvate, or prodrug thereof, in combination with a pharmaceutically acceptable excipient. Accordingly, the compounds of formula (I), (II) or (III) are useful as medicaments and/or as adjuvants for a cell therapy product, as described herein.

The present invention further relates to a compound of formula (I), (II) or (III) as defined herein for use according to the present invention, or a pharmaceutically acceptable salt, solvate, or prodrug thereof, or a pharmaceutical composition comprising any of the aforementioned entities in combination with a pharmaceutically acceptable excipient, for use in the treatment of a medical condition disclosed herein.

The compounds of formula (I), (II) or (III) or the above described pharmaceutical compositions comprising a compound of formula (I), (II) or (III) may be administered to a subject by any convenient route of administration, whether systemically/peripherally or at the site of desired action, including but not limited to one or more of: oral (e.g. as a tablet, capsule, or as an ingestible solution), topical (e.g., transdermal, intranasal, ocular, buccal, and sublingual), parenteral (e.g., using injection techniques or infusion techniques, and including, for example, by injection, e.g. subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrastemal by, e.g., implant of a depot, for example, subcutaneously or intramuscularly), pulmonary (e.g., by inhalation or insufflation therapy using, e.g., an aerosol, e.g. through mouth or nose), gastrointestinal, intrauterine, intraocular, subcutaneous, ophthalmic (including intravitreal or intracamerai), rectal, and vaginal. For example, the compounds or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

### Immunosuppressants:

In one embodiment of the invention an immunosuppressant can be employed as the endothelium damaging substance. Various immunosuppressants are known to a skilled person capable of causing endothelium damage, such as, but not limited to, those provided herein. For example, agents which are used for the treatment of inflammatory diseases are also known to have endothelium damaging side effects. Cyclosporine A leads to endothelial phenotypic changes and death (Bouvier et al., (2009), Am J Physiol Renal Physiol 296: F160-F169) and Leflunomide is an approved immunosuppressant which is known to be toxic to the endothelium (Waldman et al. (2001), Transplantation. 15;72(9):1578-82, and Zeng L et al., (2008), Transplant Proc., 40(8):2670-3).

### Treatment:

The subject or patient, such as the subject in need of treatment or prevention, may be an animal, a vertebrate animal, a mammal, a rodent (e.g. a guinea pig, a hamster, a rat, a mouse), a murine (e.g. a mouse), a canine (e.g. a dog), a feline (e.g. a cat), an equine (e.g. a horse), a primate, a simian (e.g. a monkey or ape), a monkey (e.g. a marmoset, a baboon), an ape (e. g. gorilla, chimpanzee, orangutan, gibbon), or a human. The meaning of the terms "animal", "mammal", etc. is well known in the art and can, for example, be deduced from Wehner und Gehring (1995; Thieme Verlag). In the context of this invention, it is particularly envisaged that animals are to be treated which are economically, agronomically or scientifically important. Preferably, the subject/patient is a mammal. More preferably, the subject/patient is a human.

Cancer comprises a group of diseases that can affect any part of the body and is caused by abnormal cell growth and proliferation. These proliferating cells have the potential to invade the surrounding tissue and/or to spread to other parts of the body where they form metastasis. Worldwide, there were 14 million new cases of cancer and 8.2 million cancer related deaths in 2012 (World Cancer Report 2014). The majority of cancers is caused by environmental signals involving tobacco use, obesity and infections among others, while around 5-10% are genetic cases. Cancers can be classified into subcategories based on the cell of origin. The most common subcategories are carcinomas from epithelial cells, sarcomas from connective tissue and lymphomas and leukemias from hematopoietic cells. Cancer is associated with a high variety of local and systemic symptoms and cannot be cured in many cases. In light of the high number of new cancer patients and cancer related deaths novel treatment strategies are required.

Cancer according to the present invention refers to all types of cancer or neoplasm or malignant tumors found in mammals, including leukemias, sarcomas, melanomas and carcinomas. Either solid tumors and/or liquid tumors (such as leukemia or lymphoma) may be treated.

Leukemias include, but are not limited to acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

Sarcomas include, but are not limited to a chondrosarcoma, fibrosarcoma, lymphosarcoma, melanosarcoma, myxosarcoma, osteosarcoma, Abernethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

Melanomas include, but are not limited to include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

Carcinomas include, but are not limited to acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma exulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticurn, carcinoma telangiectodes, transitional cell carcinoma, carcinoma tuberosum, tuberous carcinoma, verrucous carcinoma, and carcinoma villosum.

Additional cancers include, but are not limited to Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, esophageal cancer, genitourinary tract cancer, malignant hypercalcemia, cervical cancer, endometrial cancer, adrenal cortical cancer, and prostate cancer.

In some embodiments, "tumor" shall include, without limitation, a prostate tumor, a pancreatic tumor, a squamous cell carcinoma, a breast tumor, a melanoma, a basal cell carcinoma, a hepatocellular carcinoma, a choloangiocellular carcinoma, testicular cancer, a neuroblastoma, a glioma or a malignant astrocytic tumor such as glioblastma multiforme, a colorectal tumor, an endometrial carcinoma, a lung carcinoma, an ovarian tumor, a cervical tumor, an osteosarcoma, a rhabdo/leiomyosarcoma, a synovial sarcoma, an angiosarcoma, an Ewing sarcoma/PNET and a malignant lymphoma. These include primary tumors as well as metastatic tumors (both vascularized and non-vascularized).

As used herein, a "disease associated with inflammation" may relate to any medical condition that is commonly associated with inflammation in a subject. Inflammation as such is a phenomenon that can be identified by a skilled person using standard diagnostic approaches. Typically, the term "inflammation" as used in its art-recognized sense relates to a localized or systemic protective response elicited by injury, infection or destruction of tissues which serves to protect the subject from an injurious agent and the injured tissue. Inflammation is preferably characterized by fenestration of the microvasculature, leakage of the elements of blood into the interstitial spaces, and migration of leukocytes into the inflamed tissue, which may lead to an uncontrolled sequence of pain, heat, redness, swelling, and loss of function.

Inflammation can be classified as either acute or chronic. Acute inflammation is the initial response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. Prolonged inflammation, known as chronic inflammation, leads to a progressive shift in the type of cells present at the site of inflammation and is characterized by simultaneous destruction and healing of the tissue from the inflammatory process.

Diseases associated with inflammation may show one or more symptoms of inflammation related to at least one condition selected from the group consisting of: allergy, asthma, eczema, rheumatoid arthritis, fever, burns or other wounds, microbial infection, contact irritant dermatitis, seborrheic dermatitis, an insect bite, an insect sting, sunburn, mycosis fungoides, pyoderma gangrenosum, poison ivy, poison sumac, rosacea, ocular Inflammation, glaucoma, dry eye, red eye, ocular rosacea, blepharitis, meibomianitis, keratitis, conjunctival hyperemia, eyelid hyperemia, etc. graft-versus-host disease, chronic bronchitis, atopic dermatitis, urticaria, allergic rhinitis, allergic conjunctivitis, multiple sclerosis, tendonitis, chronic inflammation in the brain, airway inflammatory disease, inflammatory bowel disease, vernal conjunctivitis, eosinophilic granuloma, psoriasis, septic shock, ulcerative colitis, reperfusion injury of the myocardium and reperfusion injury of the brain, chronic glomerulonephritis, athlesclerosis, endotoxic shock and adult respiratory distress syndrome. In certain aspects, at least one symptom of inflammation is selected from the group consisting of: itching, edema, pain, pruritus, increased temperature, loss of function, redness, scaling, blistering, hyperpigmentation, and hypopigmentation.

The use of the endothelial damaging agent as described herein for enhancing the permeability of the blood-brain-barrier to active agents may therefore be used for the treatment of addiction, attention deficit/hyperactivity disorder (ADHD), autism, bipolar disorder, catalepsy, depression, encephalitis, epilepsy/seizures, infection, locked-in syndrome, meningitis, migraine, multiple sclerosis, myelopathy, neurodegenerative disorders, Alzheimer's, Huntington's disease, Parkinson's, Tourette's, or other medical conditions of the CNS or a neurodegenerative disease or dementia.

Neurodegenerative disease or neurodegeneration is a term for medical conditions in which the progressive loss of structure or function of neurons, including death of neurons, occurs. Many neurodegenerative diseases, including ALS, Parkinson's, Alzheimer's, and Huntington's, occur as a result of neurodegenerative processes. Such diseases are commonly considered to be incurable, resulting in progressive degeneration and/or death of neuron cells. A number of similarities are present in the features of these diseases, linking these diseases on a sub-cellular level. Some of the parallels between different neurodegenerative disorders include atypical protein assembly as well as induced cell death.

Dementia is a group of brain diseases causing a gradual decline of cognitive functions. Most of these diseases are chronic neurodegenerative diseases and are associated with neurobehavioral and/or neuropsychiatric symptoms that disable patients to independently perform activities of daily live. Alzheimer's disease is the most common form of dementia with 25 million affected individuals worldwide in the year 2000. This number is expected to increase to 114 million cases in 2050, unless preventive or neuroprotective therapy approaches emerge.

The use of the endothelial damaging agent as described herein for enhancing the permeability of the blood-brain-barrier to active agents may therefore be used for the treatment of acoustic neuroma, astrocytoma, chordoma, cns lymphoma, craniopharyngioma, glioma, brain stem glioma, ependymoma, mixed glioma, optic nerve glioma, subependymoma, medulloblastoma, meningioma, metastatic brain tumors, oligodendroglioma, pituitary tumors, primitive neuroectodermal (pnet), schwannoma, or other tumor diseases of the brain.

### Mesenchymal stem cells:

The "mesenchymal stem cells" disclosed herein are preferably cells of non-haematopoietic origin that reside in the bone marrow and other tissues. The identification and nomenclature of the MSC has been clarified by the Mesenchymal and Tissue Stem Cell Committee of the International Society for Cellular Therapy (ISCT), who have proposed a set of minimal criteria to define MSC. Typically, MSCs can give rise to connective tissue, bone, cartilage, and cells in the circulatory and lymphatic systems. Mesenchymal stem cells are found in the mesenchyme, the part of the embryonic mesoderm that consists of loosely packed, fusiform or stellate unspecialized cells. As used herein, mesenchymal stem cells include, without limitation, CD34-negative stem cells.

In one embodiment of the invention, the mesenchymal stem cells are plastic-adherent cells, defined in some embodiments as multipotent mesenchymal stromal cells and also include CD34-negative cells. For the avoidance of any doubt, the term mesenchymal stem cell encompasses multipotent mesenchymal stromal cells that also includes a subpopulation of mesenchymal cells, MSCs and their precursors, which subpopulation is made up of multipotent or pluripotent self-renewing cells capable of differentiation into multiple cell types in vivo.

As used herein, CD34-ngeative cell shall mean a cell lacking CD34, or expressing only negligible levels of CD34, on its surface. CD34-negative cells, and methods for isolating such cells, are described, for example, in Lange C. et al., "Accelerated and safe expansion of human mesenchymal stromal cells in animal serum-free medium for transplantation and regenerative medicine". J. Cell Physiol. 2007, Apr. 25.

Mesenchymal stem cells can be distinguished from hematopoietic stem cells (HSCs) by a number of indicators. For example, HSCs are known to float in culture and to not adhere to plastic surfaces. In contrast, mesenchymal stem cells adhere to plastic surfaces. The CD34-negative mesenchymal stem cells of the present invention are adherent in culture.

### Genetic modification

MSCs or any other therapeutic cell, such as the immune cells described herein, may be genetically modified to express a therapeutic gene product from an exogenous nucleic acid. As used herein, "genetically modified" shall mean any kind of cell or organism carrying modified genetic material such as nucleic acids. As used herein "nucleic acid" shall mean any nucleic acid molecule, including, without limitation, DNA, RNA and hybrids or modified variants thereof. An "exogenous nucleic acid" or "exogenous genetic element" relates to any nucleic acid introduced into the cell, which is not a component of the cells "original" or "natural" genome. Exogenous nucleic acids may be integrated or non-integrated in the genetic material of the target mesenchymal stem cell, or relate to stably transduced nucleic acids.

Any given gene delivery method is encompassed by the invention and preferably relates to viral or non-viral vectors, as well as biological or chemical methods of transfection. The methods can yield either stable or transient gene expression in the system used.

Genetically modified viruses have been widely applied for the delivery of genes into stem cells. Preferred viral vectors for genetic modification of the MSCs described herein relate to retroviral vectors, in particular to gamma retroviral vectors. The gamma retrovirus (sometimes referred to as mammalian type C retroviruses) is a sister genus to the lentivirus clade, and is a member of the Orthoretrovirinae subfamily of the retrovirus family. The Murine leukemia virus (MLV or MuLV), the Feline leukemia virus (FeLV), the Xenotropic murine leukemia virus-related virus (XMRV) and the Gibbon ape leukemia virus (GALV) are members of the gamma retrovirus genus. A skilled person is aware of the techniques required for utilization of gamma retroviruses in genetic modification of MSCs. For example, the vectors described Maetzig et al (Gammaretroviral vectors: biology, technology and application, 2001, Viruses Jun;3(6):677-713) or similar vectors may be employed. For example, the Murine Leukemia Virus (MLV), a simple gammaretrovirus, can be converted into an efficient vehicle of genetic therapeutics in the context of creating gamma retrovirus-modified MSCs and expression of a therapeutic transgene from said MSCs after delivery to a subject.

Adenoviruses may be applied, or RNA viruses such as Lentiviruses, or other retroviruses. Adenoviruses have been used to generate a series of vectors for gene transfer cellular engineering. The initial generation of adenovirus vectors were produced by deleting the EI gene (required for viral replication) generating a vector with a 4kb cloning capacity. An additional deletion of E3 (responsible for host immune response) allowed an 8kb cloning capacity. Further generations have been produced encompassing E2 and/or E4 deletions. Lentiviruses are members of Retroviridae family of viruses (M. Scherr et al., Gene transfer into hematopoietic stem cells using lentiviral vectors. Curr Gene Ther. 2002 Feb; 2(1):45-55). Lentivirus vectors are generated by deletion of the entire viral sequence with the exception of the LTRs and cis acting packaging signals. The resultant vectors have a cloning capacity of about 8 kb. One distinguishing feature of these vectors from retroviral vectors is their ability to transduce dividing and non-dividing cells as well as terminally differentiated cells.

Non-viral methods may also be employed, such as alternative strategies that include conventional plasmid transfer and the application of targeted gene integration through the use of integrase or transposase technologies. These represent approaches for vector transformation that have the advantage of being both efficient, and often site-specific in their integration. Physical methods to introduce vectors into cells are known to a skilled person. One example relates to electroporation, which relies on the use of brief, high voltage electric pulses which create transient pores in the membrane by overcoming its capacitance. One advantage of this method is that it can be utilized for both stable and transient gene expression in most cell types. Alternative methods relate to the use of liposomes or protein transduction domains. Appropriate methods are known to a skilled person and are not intended as limiting embodiments of the present invention.

In some embodiments the invention is directed to the administration of immune cells or stem cells, preferably MSCs or T cells. The prior or combined administration of an endothelial damaging agent with a cellular therapy, such as immune cells, stem cells, MSCs or T cells, can lead to an enhanced localization and enrichment in vivo of the therapeutic cells ate the site of inflammation or tumor.

### Immune cells:

Immune cells as described herein relate to biological cells involved in the immune response in a subject. Immune cells are preferably selected from T Cells, B Cells, Dendritic Cells, Granulocytes, Innate Lymphoid Cells (ILCs), Megakaryocytes, Monocytes/Macrophages, Natural Killer (NK) Cells, Platelets, Red Blood Cells(RBCs) and/or Thymocytes.

T cells or T lymphocytes are a type of lymphocyte (a subtype of white blood cell) that play a central role in cell-mediated immunity. They can be distinguished from other lymphocytes, such as B cells and natural killer cells, by the presence of a T-cell receptor on the cell surface. The several subsets of T cells each have a distinct function. T cell subtypes include, without limitation, T helper type 1 (Th1) cells, T helper type 2 (Th2) cells, T helper type 9 (Th9) cells, T helper type 17 (Th17) cells, T helper type 22 (Th22) cells, Follicular helper T (Tfh) cells, Regulatory T (Treg) cells, Natural killer T (NKT) cells, Gamma delta T cells, CD8+ cytotoxic T lymphocytes (CTLs). Further non-limiting embodiments of T cells include thymocytes, immature T lymphocytes, mature T lymphocytes, resting T lymphocytes, cytokine-induced killer cells (CIK cells) or activated T lymphocytes. Cytokine-induced killer (CIK) cells are typically CD3- and CD56-positive, non-major histocompatibility complex (MHC)-restricted, natural killer (NK)-like T lymphocytes. The T cell can be a CD4+ T cell, a cytotoxic T cell (CTL; CD8+ T cell), CD4+CD8+ T cell, CD4 CD8 T cell, or any other subset of T cells.

A T cell can be engineered with defined specificity by artificial T cell receptors (also known as chimeric T cell receptors, chimeric immunoreceptors, chimeric antigen receptors (CARs)) which are composite molecules and graft an arbitrary specificity onto an immune effector cell and activate the engineered cell upon engagement of cognate target. Typically, these receptors are used to graft the specificity of a monoclonal antibody onto a T cell linked to an intracellular signaling domain for activation.

Genetically engineered T cells may be created by infecting patient's cells with a retro- or lentivirus encoding a T cell receptor (TCR) or a CAR that is specific to recognize tumor antigens. Once the T cell binds to its target antigen, the stimulatory molecules provide the necessary signals for the T cell to become fully active. In this fully active state, the T cells can more effectively proliferate and can attack cancer cells.

For example, when reintroduced back to patients after autologous cell transplantation, the T cells modified with the CAR of the invention as described herein may recognize and kill tumor cells. CIK cells may have enhanced cytotoxic activity compared to other T cells, and therefore represent a preferred embodiment of an immune cell of the present invention. As would be understood by the skilled person, other cells may also be used as immune effector cells with the CARs as described herein. In particular, immune effector cells also include NK cells, NKT cells, neutrophils, and macrophages. Immune effector cells also include progenitors of effector cells wherein such progenitor cells can be induced to differentiate into an immune effector cells in vivo or in vitro.

CARs are typically composed of an extracellular ectodomain derived from an antibody and an endodomain comprising signaling modules derived from T cell signaling proteins. In a preferred embodiment, the ectodomain preferably comprises variable regions from the heavy and light chains of an immunoglobulin configured as a single-chain variable fragment (scFv). The scFv is preferably attached to a hinge region that provides flexibility and transduces signals through an anchoring transmembrane moiety to an intracellular signaling domain. The transmembrane domains originate preferably from either CD8α or CD28. In the first generation of CARs the signaling domain consists of the zeta chain of the TCR complex. The term "generation" refers to the structure of the intracellular signaling domains. Second generation CARs are equipped with a single costimulatory domain originated from CD28 or 4-1BB. Third generation CARs already include two costimulatory domains, e.g. CD28, 4-1 BB, ICOS or OX40, CD3 zeta.

In various embodiments, genetically engineered receptors that redirect cytotoxicity of immune effector cells toward cancer cells are provided. These genetically engineered receptors referred to herein as chimeric antigen receptors (CARs). CARs are molecules that combine antibody-based specificity for a desired antigen (e.g., a tumor-associated antigen) with a T cell receptor-activating intracellular domain to generate a chimeric protein that exhibits a specific anti-BCMA cellular immune activity. As used herein, the term, "chimeric," describes being composed of parts of different proteins or DNAs from different origins.

CARs contemplated herein, comprise an extracellular domain (also referred to as a binding domain or antigen-binding domain) that binds to a tumor-associated antigen, a transmembrane domain, and an intracellular domain, or intracellular signaling domain. Engagement of the anti-BCMA antigen binding domain of the CAR with BCMA on the surface of a target cell results in clustering of the CAR and delivers an activation stimulus to the CAR-containing cell. The main characteristic of CARs are their ability to redirect immune effector cell specificity, thereby triggering proliferation, cytokine production, phagocytosis or production of molecules that can mediate cell death of the target antigen expressing cell in a major histocompatibility (MHC) independent manner, exploiting the cell specific targeting abilities of monoclonal antibodies, soluble ligands or cell specific co-receptors.

In various embodiments, a CAR comprises an extracellular binding domain that comprises a humanized tumor-antigen-specific binding domain; a transmembrane domain; one or more intracellular signaling domains. In particular embodiments, a CAR comprises an extracellular binding domain that comprises a humanized anti-tumor-antigen antigen binding fragment thereof; one or more spacer domains; a transmembrane domain; one or more intracellular signaling domains.

The "extracellular antigen-binding domain" or "extracellular binding domain" provide a CAR with the ability to specifically bind to the target antigen of interest. The binding domain may be derived either from a natural, synthetic, semi-synthetic, or recombinant source. Preferred are scFV domains.

"Specific binding" is to be understood as via one skilled in the art, whereby the skilled person is clearly aware of various experimental procedures that can be used to test binding and binding specificity. Methods for determining equilibrium association or equilibrium dissociation constants are known in the art. Some cross-reaction or background binding may be inevitable in many protein-protein interactions; this is not to detract from the "specificity" of the binding between CAR and epitope. "Specific binding" describes binding of an anti-tumor-antigen antibody or antigen binding fragment thereof (or a CAR comprising the same) to a tumor antigen at greater binding affinity than background binding. The term "directed against" is also applicable when considering the term "specificity" in understanding the interaction between antibody and epitope.

An "antigen (Ag)" refers to a compound, composition, or substance that can stimulate the production of antibodies or a T cell response in an animal. In particular embodiments, the target antigen is a tumor-associated antigen, in other words an epitope of a polypeptide expressed predominantly in tumor cells. An "epitope" refers to the region of an antigen to which a binding agent binds. Epitopes can be formed both from contiguous amino acids or noncontiguous amino acids juxtaposed by tertiary folding of a protein.

"Single-chain Fv" or "scFv" antibody fragments comprise the VH and VL domains of antibody, wherein these domains are present in a single polypeptide chain and in either orientation {e.g., VL-VH or VH-VL). Generally, the scFv polypeptide further comprises a polypeptide linker between the VH and VL domains which enables the scFv to form the desired structure for antigen binding. In preferred embodiments, a CAR contemplated herein comprises antigen-specific binding domain that is an scFv and may be a murine, human or humanized scFv. Single chain antibodies may be cloned form the V region genes of a hybridoma specific for a desired target. In particular embodiments, the antigen-specific binding domain that is a humanized scFv that binds a human tumor-antigen polypeptide.

Cellular therapies typically involve the administration of immune cells isolated from the blood or from a tumor of the patient. Immune cells directed towards the tumor to be treated are activated, cultured and returned to the patient where the immune cells attack the cancer. Cell types that can be used in this way are, without limitation, natural killer cells, lymphokine-activated killer cells, cytotoxic T cells, monocytes, macrophages, granulocytes and dendritic cells. Dendritic cell therapy provokes anti-tumor responses by causing dendritic cells to present tumor antigens. Dendritic cells present antigens to lymphocytes, which activates them, priming them to kill other cells that present the antigen.

One approach to these previous attempts at immunotherapy involves engineering patients' own immune cells to recognize and attack their tumors. And although this approach, called adoptive cell transfer (ACT), has been restricted to clinical trials so far, treatments using these engineered immune cells have generated therapeutic responses in patients with advanced cancer.

Dendritic cells (DCs) are antigen-presenting cells of the mammalian immune system. Their main function is to process antigen material and present it on the cell surface to the T cells of the immune system. They act as messengers between the innate and the adaptive immune systems. Dendritic cells are present in those tissues that are in contact with the external environment, such as the skin (where there is a specialized dendritic cell type called the Langerhans cell) and the inner lining of the nose, lungs, stomach and intestines. They can also be found in an immature state in the blood. Once activated, they migrate to the lymph nodes where they interact with T cells and B cells to initiate and shape the adaptive immune response. Owing to their properties, dendritic cells have become the natural agents for antigen delivery and vaccination strategies involving DCs have been developed. The aim of DC vaccination is to induce tumor-specific effector T cells that can reduce the tumor mass specifically and that can induce immunological memory to control tumor relapse. In this process, the first step is to provide DCs with tumor-specific antigens. This can be achieved by culturing ex vivo DCs that have been derived from patients with an adjuvant (that induces DC maturation) and the tumor-specific antigen, and then injecting these cells back into the patient, or by inducing DCs to take up the tumor-specific antigen in vivo. To improve the therapeutic use of DC vaccination strategies the co-administration of DCs together with MSC according the present invention is envisioned.

Monocytes are a type of white blood cells (leukocytes). They are the largest of all leukocytes. They are part of the innate immune system of vertebrates including all mammals (humans included). Monocytes are produced by the bone marrow from precursors called monoblasts, bipotent cells that differentiated from hematopoietic stem cells. Monocytes circulate in the bloodstream for about one to three days and then typically move into tissues throughout the body. They constitute between three to eight percent of the leukocytes in the blood. In tissues monocytes mature into different types of macrophages at different anatomical locations.

Macrophages, sometimes called macrophagocytes, are cells produced by the differentiation of monocytes in tissues. Monocytes and macrophages are phagocytes. Macrophages function in both non-specific defense (innate immunity) as well as help initiate specific defense mechanisms (adaptive immunity) of vertebrate animals. Macrophages predominantly expressing the killer phenotype are called M1 macrophages, whereas those involved in tissue repair are called M2 macrophages. The role of macrophages is to phagocytose, or engulf and then digest, cellular debris and pathogens, either as stationary or as mobile cells. They also stimulate lymphocytes and other immune cells to respond to pathogens (Palucka K et al. Nat Rev Cancer. 2012 Mar 22;12(4):265-77. doi: 10.1038/nrc3258). They are specialized phagocytic cells that attack foreign substances, infectious microbes and cancer cells through destruction and ingestion.

Innate lymphoid cells (ILCs) are a group of innate immune cells that belong to the lymphoid lineage (lymphocytes) but do not respond in an antigen-specific manner, as they lack a B or T cell receptor. This relatively newly described group of cells has different physiological functions, some of them analogous to helper T cells, while also including the cytotoxic NK cells. In accordance, they have an important role in protective immunity and the regulation of homeostasis and inflammation. Natural killer (NK) cells are cytotoxic innate effector cells analogous to the cytotoxic T cells of the adaptive immune system. They are distributed throughout the blood, organs, and lymphoid tissue and make up around 15% of the peripheral blood lymphocytes. NK cells play a role in tumor surveillance and the rapid elimination of virus-infected cells. They do not require the missing "self" signal of MHC Class I and can recognize stressed cells in the absence of antibodies, allowing them to react much more quickly than the adaptive immune system. Natural killer (NK) cells play critical roles in host immunity against cancer. In response, cancers develop mechanisms to escape NK cell attack or induce defective NK cells (Cheng M et al. Cell Mol Immunol. 2013 May;10(3):230-52. doi: 10.1038/cmi.2013.10. Epub 2013 Apr 22.). Current NK cell-based cancer immunotherapy aims to overcome NK cell paralysis using several approaches and the combined administration of MSCs might help to make NK cell based therapies more effective.

Granulocytes are a category of white blood cells characterized by the presence of granules in their cytoplasm encompassing neutrophils, eosinophils, basophils and mast cells.

The present invention therefore encompasses adoptive cell transfer in combination with administration of the MSCs described herein. It is encompassed within the invention that administration of MSCs prior or simultaneous to immune cells (e.g. CAR-Ts) will enhance the anti-tumor activity of CAR-Ts, TCR-transgeneic T-cells, in vitro expanded T Cells or other immune effector cells administered during adoptive cell transfer. The endothelium damaging agent will therefore also improve enrichment of the therapeutic cells at the site of interest. The presence of the MSC will enhances the activation of T-cells only locally, preferably within or in proximity to the tumor, and subsequently lead to a memory effector cell phenotype, thereby prolonging the therapeutic effect of the treatment.

### Administration

The present invention encompasses treatment of a patient by introducing a therapeutically effective number of cells into a subject's bloodstream. As used herein, "introducing" cells "into the subject's bloodstream" shall include, without limitation, introducing such cells into one of the subject's veins or arteries via injection. Such administering can also be performed, for example, once, a plurality of times, and/or over one or more extended periods. A single injection is preferred, but repeated injections over time (e.g., weekly, monthly, quarterly, half-yearly or yearly) may be necessary in some instances. Such administering is also preferably performed using an admixture of CD34-negative cells and a pharmaceutically acceptable carrier. Pharmaceutically acceptable carriers are well known to those skilled in the art and include, but are not limited to, 0.01-0.1 M and preferably 0.05 M phosphate buffer or 0.8% saline, as well as commonly used proprietary cryopreservation media.

Administration may also occur locally, for example by injection into an area of the subject's body in proximity to a tumor disease. MSCs have been shown to migrate towards cancerous tissue. Regardless, the local administration of the cells as described herein may lead to high levels of the cells at their site of action.

Additionally, such pharmaceutically acceptable carriers can be aqueous or non-aqueous solutions, suspensions, and emulsions, most preferably aqueous solutions. Aqueous carriers include water, alcoholic/aqueous solutions, emulsions and suspensions, including saline and buffered media. Parenteral vehicles include sodium chloride solution, Ringer's dextrose, dextrose and sodium chloride, lactated Ringer's and fixed oils. Intravenous vehicles include fluid and nutrient replenishers, electrolyte replenishers such as Ringer's dextrose, those based on Ringer's dextrose, and the like. Fluids used commonly for i.v. administration are found, for example, in Remington: The Science and Practice of Pharmacy, 20th Ed., p. 808, Lippincott Williams S-Wilkins (2000). Preservatives and other additives may also be present, such as, for example, antimicrobials, antioxidants, chelating agents, inert gases, and the like.

As used herein, a "therapeutically effective number of cells" includes, without limitation, the following amounts and ranges of amounts: (i) from about 1 x 10² to about 1 x 10⁸ cells/kg body weight; (ii) from about 1 x 10³ to about 1 x 10⁷ cells/kg body weight; (iii) from about 1 x 10⁴ to about 1 x 10⁶ cells/kg body weight; (iv) from about 1 x 10⁴ to about 1 x 10⁵ cells/kg body weight; (v) from about 1 x 10⁵ to about 1 x 10⁶ cells/kg body weight; (vi) from about 5 x 10⁴ to about 0.5 x 10⁵ cells/kg body weight; (vii) about 1 x 10³ cells/kg body weight; (viii) about 1 x 10⁴ cells/kg body weight; (ix) about 5 x 10⁴ cells/kg body weight; (x) about 1 x 10⁵ cells/kg body weight; (xi) about 5 x 10⁵ cells/kg body weight; (xii) about 1 x 10⁶ cells/kg body weight; and (xiii) about 1 x 10⁷ cells/kg body weight. Human body weights envisioned include, without limitation, about 5 kg, 10 kg, 15 kg, 30 kg, 50 kg, about 60 kg; about 70 kg; about 80 kg, about 90 kg; about 100 kg, about 120 kg and about 150 kg. These numbers are based on pre-clinical animal experiments and human trials and standard protocols from the transplantation of CD34+ hematopoietic stem cells. Mononuclear cells (including CD34+ cells) usually contain between 1:23000 to 1:300000 CD34-negative cells.

As used herein, "treating" a subject afflicted with a disorder shall mean slowing, stopping or reversing the disorder's progression. In the preferred embodiment, treating a subject afflicted with a disorder means reversing the disorder's progression, ideally to the point of eliminating the disorder itself. As used herein, ameliorating a disorder and treating a disorder are equivalent. The treatment of the present invention may also, or alternatively, relate to a prophylactic administration of said cells. Such a prophylactic administration may relate to the prevention of any given medical disorder, or the prevention of development of said disorder, whereby prevention or prophylaxis is not to be construed narrowly under all conditions as absolute prevention. Prevention or prophylaxis may also relate to a reduction of the risk of a subject developing any given medical condition, preferably in a subject at risk of said condition.

A combined therapy may precede or follow treatment with therapeutic cells, by intervals ranging from minutes to weeks. In embodiments where the other immunotherapeutic agent and the MSCs are administered separately to the site of interest, one would generally ensure that a significant period of time did not expire between the time of each delivery, such that the agent and the MSCs would still be able to exert an advantageously combined effect on a treatment site. In such instances, it is contemplated that one would contact the subject with both modalities within about 12-24 h of each other and, more preferably, within about 6-12 h of each other, with a delay time of only about 12 h being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

The subject from which the mesenchymal stem cells and/or immune cells are obtained may be the same subject, to whom the cells are intended to be administered after cultivation. Such cells may therefore be considered autologous. The cells may however be obtained from a subject distinct from the intended patient, therefore being considered allogenic. As used herein, a cell is "allogenic" with respect to a subject if it or any of its precursor cells are from another subject of the same species. As used herein, a cell is "autologous" with respect to a subject if it or its precursor cells are from the same subject. In a preferred embodiment, the immune cells are autologous to the subject of medical treatment.

### Further definitions:

The term "stroma" as used herein refers to the supportive framework of a tissue or an organ (or gland, tissue or other structure), usually composed of extracellular matrix (ECM) and stromal cells. The stroma is distinct from the parenchyma, which consists of the key functional elements of that organ. Stromal cells (in the dermis layer) adjacent to the epidermis (the very top layer of the skin) release growth factors that promote cell division. Stroma is made up of the non-malignant host cells. Stroma provides an extracellular matrix on which tumors can grow or maintain existence or separate themselves from the immune environment.

As used herein, the term "tumor microenvironment" relates to the cellular environment in which any given tumor exists, including the tumor stroma, surrounding blood vessels, immune cells, fibroblasts, other cells, signalling molecules, and the ECM.

As used herein "cell migration" or "homing" is intended to mean movement of a cell towards a particular chemical or physical signal. Cells often migrate in response to specific external signals, including chemical signals and mechanical signals. The MSCs as described herein are capable of homing to tumor tissue or other inflammation signals.

Chemotaxis is one example of cell migration regarding response to a chemical stimulus. In vitro chemotaxis assays such as Boyden chamber assays may be used to determine whether cell migration occurs in any given cell. For example, the cells of interest may be purified and analysed. Chemotaxis assays (for example according to Falk et al., 1980 J. Immuno. Methods 33:239-247) can be performed using plates where a particular chemical signal is positioned with respect to the cells of interest and the transmigrated cells then collected and analysed. For example, Boyden chamber assays entail the use of chambers isolated by filters, used as tools for accurate determination of chemotactic behaviour. The pioneer type of these chambers was constructed by Boyden (Boyden (1962) "The chemotactic effect of mixtures of antibody and antigen on polymorphonuclear leucocytes". J Exp Med 115 (3): 453). The motile cells are placed into the upper chamber, while fluid containing the test substance is filled into the lower one. The size of the motile cells to be investigated determines the pore size of the filter; it is essential to choose a diameter which allows active transmigration. For modelling in vivo conditions, several protocols prefer coverage of filter with molecules of extracellular matrix (collagen, elastin etc.) Efficiency of the measurements can be increased by development of multiwell chambers (e.g. NeuroProbe), where 24, 96, 384 samples are evaluated in parallel. Advantage of this variant is that several parallels are assayed in identical conditions.

As used herein "engraftment" relates to the process of incorporation of grafted or transplanted tissue or cells into the body of the host. Engraftment may also relate to the integration of transplanted cells into host tissue upon administration and their survival and under some conditions differentiation into non-stem cell states.

Techniques for assessing engraftment, and thereby to some extent both migration and the biodistribution of MSCs, can encompass either in vivo or ex vivo methods. Examples of in vivo methods include bioluminescence, whereby cells are transduced to express luciferase and can then be imaged through their metabolism of luciferin resulting in light emission; fluorescence, whereby cells are either loaded with a fluorescent dye or transduced to express a fluorescent reporter which can then be imaged; radionuclide labelling, where cells are loaded with radionuclides and localized with scintigraphy, positron emission tomography (PET) or single photon emission computed tomography (SPECT); and magnetic resonance imaging (MRI), wherein cells loaded with paramagnetic compounds (e.g., iron oxide nanoparticles) are traced with an MRI scanner. Ex vivo methods to assess biodistribution include quantitative PCR, flow cytometry, and histological methods. Histological methods include tracking fluorescently labelled cells; in situ hybridization, for example, for Y-chromosomes and for human-specific ALU sequences; and histochemical staining for species-specific or genetically introduced proteins such as bacterial β-gatactosidase. These immunohistochemical methods are useful for discerning engraftment location but necessitate the excision of tissue. For further review of these methods and their application see Kean et al., MSCs: Delivery Routes and Engraftment, Cell-Targeting Strategies, and Immune Modulation, Stem Cells International, Volume 2013 (2013).

As used herein, in "proximity with" a tissue includes, for example, within 50 mm, 10 mm, 5 mm, within 1 mm of the tissue, within 0.5 mm of the tissue and within 0.25 mm of the tissue.

Transport of cells or other agents across the BBB or in the brain can be followed and assessed using quantitative or semi-quantitative imagine techniques, for example neural optical imaging, such as is discussed in Aswendt et al., (Front Cell Neurosci. 2014; 8: 226), which discloses a number of suitable optical methods for following BBB permeation of cellular therapeutics. Methods can be employed that permit simple animal preparation, repetitive experimental conditions, relatively medium-cost instrumentation and are performed under mild anesthesia, thus nearly under physiological conditions. Optical intrinsic imaging, fluorescence imaging and bioluminescence imaging to characterize pathology and transport, homing and/or engraftment of stem cells can be used.

Non-cellular therapeutic agents for combined administration with the endothelial damaging agent, to be transported across the BBB, relate to any agent with a therapeutic effect in treating a disease of the CNS or brain, such as those described herein.

### FIGURES

The following figures are presented in order to describe particular embodiments of the invention, by demonstrating a practical implementation of the invention, without being limiting to the scope of the invention or the concepts described herein.

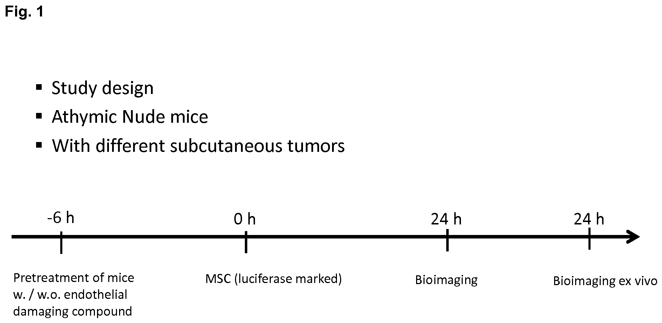

**Fig. 1****:** Graphical representation of the study design to test endothelial damaging compounds in murine tumor models:
   1411HP tumor cells (8x10^6 cells per mouse) were injected subcutaneously in to athymic nude mice. Tumor formation was followed over 17-20 days, treatment was initiated when the tumors reached a volume of more than 150mm^3. Mice were either treated with a combretastatin derivative (1-Methyl-5-(3-amino-4-ethoxyphenyl)-4-(3-bromo-4,5-dimethoxyphenyl)-imidazol bis(hydrochlorid)) according to WO 2011/138409 (25mg/kg, i.p.) or treated with PBS. 6h later the mice received i.v. 3x10e6 human mesenchymal stem cells. The mesenchymal stem cells were genetically engineered to express luciferase. 24h after the MSC treatment mice were treated with luciferin (i.p.) and afterwards imaged using an bioimaging system (IVIS) to detect luciferase signals in vivo. Shortly afterwards mice were sacrificed and the organs and tumors were collected. Organs and tumors were imaged again for luciferase activity ex vivo. The results indicate that pretreatment with the endothelial damaging combretastatin analogue increases the the tumor specific enrichment of the MSC compared to untreated mice.

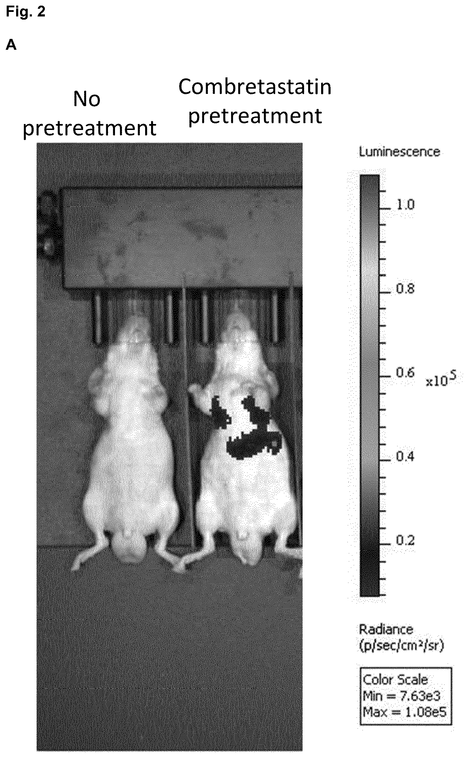

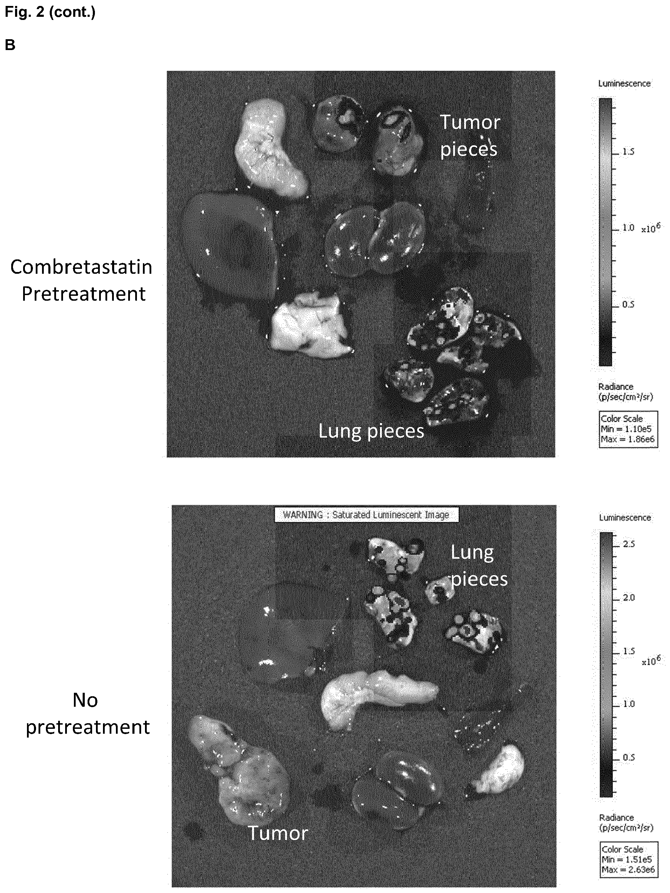

**Fig. 2****:** MSC show enhanced in vivo enrichment in 1411HP tumors after pretreating with an combretastatin analogue:
   1411HP tumor cells (8x10^6 cells per mouse) were injected subcutaneously in to athymic nude mice. Tumor formation was followed over 17-20 days, treatment was initiated when the tumors reached a volume of more than 150mm^3. Mice were either treated with a combretastatin derivative (1-Methyl-5-(3-amino-4-ethoxyphenyl)-4-(3-bromo-4,5-dimethoxyphenyl)-imidazol bis(hydrochlorid)) according to WO 2011/138409 (25mg/kg, i.p.) or treated with PBS. 6h later the mice received i.v. 3x10e6 human mesenchymal stem cells. The mesenchymal stem cells were genetically engineered to express luciferase. 24h after the MSC treatment mice were treated with luciferin (i.p.) and afterwards imaged using an bioimaging system (IVIS) to detect luciferase signals in vivo. Shortly afterwards mice were sacrificed and the organs and tumors were collected. Organs and tumors were imaged again for luciferase activity ex vivo. The results indicate that pretreatment with the endothelial damaging combretastatin analogue increases the the tumor specific enrichment of the MSC compared to untreated mice.
   Figure 2A depicts the in vivo bioluminescence signal. Pretreated mice show a stronger signal in the lung than untreated mice.
   Figure 2B depicts the ex vivo signals in the organs and tumors. Pretreated mice show strong signals of MSC in the tumor. Mice without pretreatment do not show any signs of MSC presence in the tumor.
**Fig. 3****:** MSC show enhanced in vivo enrichment in DLD1 tumors after pretreating with an combretastatin analogue:
   DLD1 tumor cells (5x10^6 cells per mouse) were injected subcutaneously in to athymic nude mice. Tumor formation was followed over 9-11 days, treatment was initiated when the tumors reached a volume of more than 150mm^3. Mice were either treated with a combretastatin derivative (1-Methyl-5-(3-amino-4-ethoxyphenyl)-4-(3-bromo-4,5-dimethoxyphenyl)-imidazol bis(hydrochlorid)) according to WO 2011/138409 (25mg/kg, i.p.) or treated with PBS. 6h later the mice received i.v. 3x10e6 human mesenchymal stem cells. The mesenchymal stem cells were genetically engineered to express luciferase. 24h after the MSC treatment mice were treated with luciferin (i.p.) and afterwards imaged using an bioimaging system (IVIS) to detect luciferase signals in vivo. Shortly afterwards mice were sacrificed and the organs and tumors were collected. Organs and tumors were imaged again for luciferase activity ex vivo. The results indicate that pretreatment with the endothelial damaging combretastatin analogue increases the the tumor specific enrichment of the MSC compared to untreated mice.

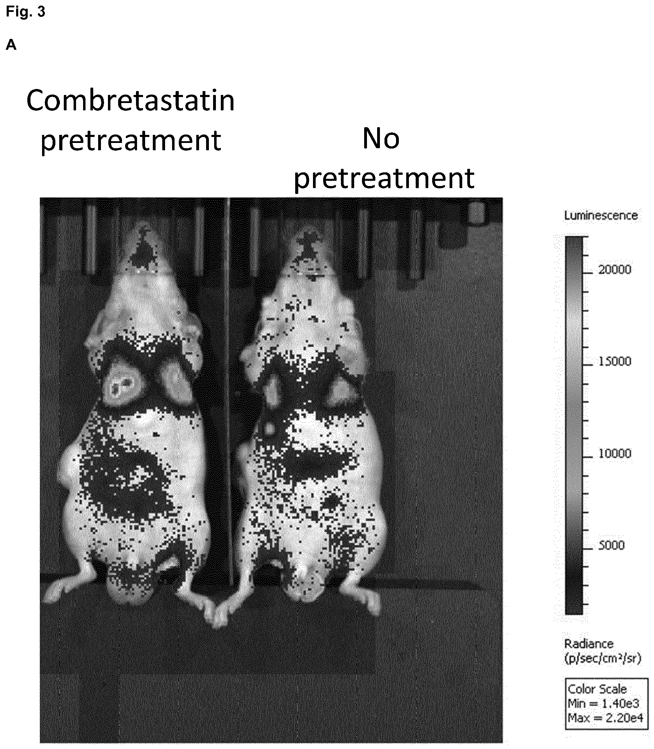

   Figure 3A depicts the in vivo bioluminescence signal. Pretreated mice show a stronger signal in the lung than untreated mice.

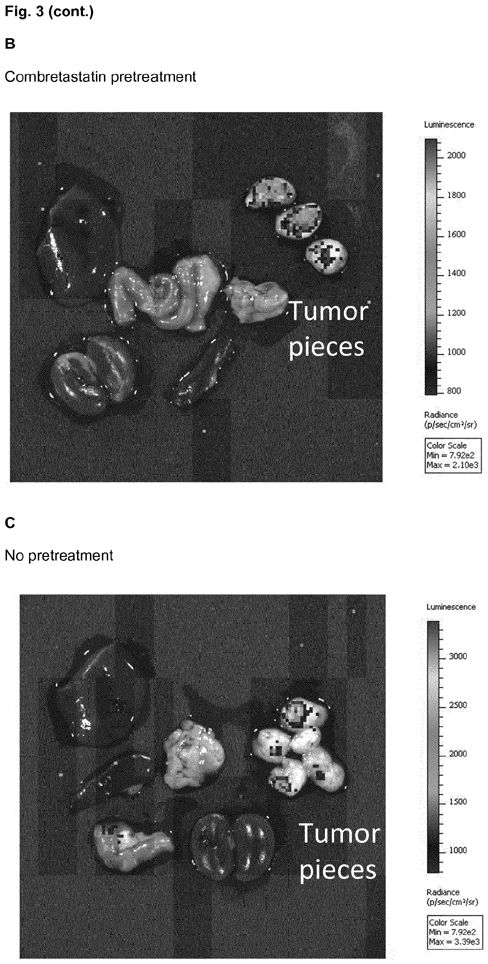

   Figure 3B depicts the ex vivo signals in the organs and tumors after combretastatin analogue pretreatment. Pretreated mice show strong signals of MSC in the tumor.
   Figure 3C depicts the ex vivo signals in the organs and tumors after no pretreatment. Mice without pretreatment only show very weak signs of MSC presence in the tumor.
**Fig. 4****:** HUVEC network assay.

Appearance of HUVEC networks in type I calf skin collagen, 4 h after treatment. (A) Control (incubated with media containing 1% DMSO), (B) treated with combretastatin A-4 (0.31 mM) in DMSO, breakdown of network is visible (K Grosios et al., British Journal of Cancer (1999) 81(8), 1318-1327).

### EXAMPLES

The following examples are presented in order to describe practical and in some cases preferred embodiments of the invention, by demonstrating a practical implementation of the invention, without being limiting to the scope of the invention or the concepts described herein.

### Preparation of human mesenchymal stem cells

The MSC were prepared according to the apceth process, for example as described using the cell culture medium disclosed in US8557578 B2. The cells were isolated from bone marrow from healthy human donors by plastic adherence and are cultured in growth medium.

Human MSCs are isolated from bone marrow by plastic adherence and are cultured in growth medium e.g. FBS containing DMEM as described by Pittinger, M.F. (2008) Mesenchymal stem cells from adult bone marrow, In D.J. Prockop, D.G. Phinney, B.A. Bunnell, Methods in Molecular Biology 449, Mesenchymal stem cells, Totowa: Humana Press), or in the culture medium as described in US8557578 B2.

### Genetic modification of mesenchymal stem cell:

The transduction is performed with modifications as described by Murray et al., 1999 Human Gene Therapy. 10(11): 1743-1752 and Davis et al., 2004 Biophysical Journal Volume 86 1234-1242. In detail:
6-well cell culture plates (e.g. Corning) are coated with Poly-L-Lysine (PLL) (e.g. Sigma-Aldrich, P4707-50ML): The PLL solution (0.01%) is diluted to final concentration between 0.0001% and 0.001% with PBS. 2ml of the diluted PLL are used for each well. The plate is incubated at least for 2h at room temperature. After incubation, the plates are washed carefully with PBS.

Viral vector supernatant in a final volume of 2ml is added to each PLL-coated well. The number of particles should between 2x10e3 and 1x10e6 per well, which will result in multiplicity of infection of 0.25 and 10. The loaded plate is centrifuged for 2000x g, 30min, 4°C. Afterwards the supernatant is discarded and 1x10e5 mesenchymal stem cells are seeded per well. The plates are incubated at 37° with 5% CO2 for further use.

**The remaining examples are provided as references to the figures, in which the surprising effect of an endothelium damaging agent is described, which enhances the tumor-specific enrichment of mesenchymal stem cells after systemic application.**

## Claims

1. Cell therapy product comprising mammalian therapeutic cells for use in the treatment of a solid tumor disease, a disease associated with inflammation or a disorder of the central nervous system (CNS) or neurological or neurodegenerative disorder, wherein the use of said cell therapy product comprises administration of a vascular endothelial damaging agent prior to or in combination with administration of said cell therapy product.

2. Cell therapy product for the use according to claim 1, wherein the vascular endothelial damaging agent is administered as an adjuvant in an amount and/or in a manner effective to enhance the enrichment of said therapeutic cells at a target site in the subject of treatment, preferably wherein the cell therapy product is administered systemically, preferably intravenously, intra-arterially and/or intraperitoneally.

3. Cell therapy product for the use according to the preceding claim, wherein the vascular endothelial damaging agent is a combretastatin or an analogue or derivative thereof.

4. Cell therapy product for the use according to the preceding claim, wherein the combretastatin analogue or derivative is 1-Methyl-5-(3-amino-4-ethoxyphenyl)-4-(3-bromo-4,5-dimethoxyphenyl)-imidazol bis(hydrochlorid) or 1-Methyl-5-(3-amino-4-methoxyphenyl)-4-(3-bromo-4,5-dimethoxyphenyl)-imidazol bis(hydrochlorid).

5. Cell therapy product for the use according to any one of the preceding claims, wherein the cell therapy product is administered from 0.5 to 72 hours, preferably 1 to 24 hours, more preferably 2 to 12 hours after administration of said vascular endothelial damaging agent.

6. Cell therapy product for the use according to any one of the preceding claims, wherein the cell therapy product comprises or consists of mesenchymal stem cells (MSCs).

7. Cell therapy product for the use according to the preceding claim, wherein the MSCs are genetically modified to express a therapeutic gene product from an exogenous nucleic acid.

8. Cell therapy product for the use according to any one of claims 1 to 5, wherein the cellular therapy product comprises or consists of T cells.

9. Cell therapy product for the use according to the preceding claim, wherein the T cells are chimeric antigen receptor T cells (CAR-Ts).

10. Cell therapy product for the use according to any one of the preceding claims, wherein one or more cell therapy products comprising MSCs and CAR-Ts are administered in combination.

11. Composition comprising a cell therapy product comprising mammalian therapeutic cells and a vascular endothelial damaging agent for use as a medicament.

12. Composition according to the preceding claim, wherein the vascular endothelial damaging agent is a combretastatin or an analogue or derivative thereof and the cell therapy product comprises or consists of mesenchymal stem cells (MSCs) and/or T cells.

13. Combination of a cell therapy product comprising mammalian therapeutic cells and a vascular endothelial damaging agent, wherein said cell therapy product and vascular endothelial damaging agent are packaged together for immediate combined use as a medicament.

14. Combination according to the preceding claim, wherein the vascular endothelial damaging agent is a combretastatin or an analogue or derivative thereof and the cell therapy product comprises or consists of mesenchymal stem cells (MSCs) and/or T cells.

## Patentansprüche

1. Zelltherapieprodukt, umfassend therapeutische Säugetierzellen zur Verwendung bei der Behandlung einer soliden Tumorerkrankung, einer mit einer Entzündung oder einer Störung des Zentralnervensystems (ZNS) verbundenen Krankheit oder einer neurologischen oder neurodegenerativen Störung, wobei die Verwendung des Zelltherapieprodukts die Verabreichung eines gefäßendothelschädigenden Mittels vor oder in Kombination mit der Verabreichung des Zelltherapieprodukts umfasst.

2. Zelltherapieprodukt zur Verwendung nach Anspruch 1, wobei das gefäßendothelschädigende Mittel als Adjuvans in einer Menge und/oder in einer Weise verabreicht wird, die wirksam ist, um die Anreicherung der therapeutischen Zellen an einer Zielstelle im behandelten Patienten zu verbessern, vorzugsweise wobei das Zelltherapieprodukt systemisch, vorzugsweise intravenös, intraarteriell und/oder intraperitoneal verabreicht wird.

3. Zelltherapieprodukt zur Verwendung nach dem vorhergehenden Anspruch, wobei das gefäßendothelschädigende Mittel ein Combretastatin oder ein Analogon oder Derivat davon ist.

4. Zelltherapieprodukt zur Verwendung nach dem vorhergehenden Anspruch, wobei das Combretastatin-Analogon oder -Derivat 1-Methyl-5-(3-amino-4-ethoxyphenyl)-4-(3-brom-4,5-dimethoxyphenyl)-imidazol-bis(hydrochlorid) oder 1-Methyl-5-(3-amino-4-methoxyphenyl)-4-(3-brom-4,5-dimethoxyphenyl)-imidazol-bis(hydrochlorid) ist.

5. Zelltherapieprodukt zur Verwendung nach einem der vorhergehenden Ansprüche, wobei das Zelltherapieprodukt von 0,5 bis 72 Stunden, vorzugsweise 1 bis 24 Stunden, bevorzugter 2 bis 12 Stunden nach Verabreichung des gefäßendothelschädigenden Mittels verabreicht wird.

6. Zelltherapieprodukt zur Verwendung nach einem der vorhergehenden Ansprüche, wobei das Zelltherapieprodukt mesenchymale Stammzellen (MSZ) umfasst oder daraus besteht.

7. Zelltherapieprodukt zur Verwendung nach dem vorhergehenden Anspruch, wobei die MSZ genetisch modifiziert sind, um ein therapeutisches Genprodukt aus einer exogenen Nukleinsäure zu exprimieren.

8. Zelltherapieprodukt zur Verwendung nach einem der Ansprüche 1 bis 5, wobei das Zelltherapieprodukt T-Zellen umfasst oder daraus besteht.

9. Zelltherapieprodukt zur Verwendung nach dem vorhergehenden Anspruch, wobei die T-Zellen chimäre Antigen-Rezeptor-T-Zellen (CAR-T) sind.

10. Zelltherapieprodukt zur Verwendung nach einem der vorhergehenden Ansprüche, wobei ein oder mehrere Zelltherapieprodukte, die MSZ und CAR-T umfassen, in Kombination verabreicht werden.

11. Zusammensetzung, umfassend ein Zelltherapieprodukt, das therapeutische Säugetierzellen und ein gefäßendothelschädigendes Mittel zur Verwendung als Medikament umfasst.

12. Zusammensetzung nach dem vorhergehenden Anspruch, wobei das gefäßendothelschädigende Mittel ein Combretastatin oder ein Analogon oder Derivat davon ist und das Zelltherapieprodukt mesenchymale Stammzellen (MSZ) und/oder T-Zellen umfasst oder daraus besteht.

13. Kombination eines Zelltherapieprodukts, das therapeutische Säugetierzellen umfasst, und eines gefäßendothelschädigenden Mittels, wobei das Zelltherapieprodukt und das gefäßendothelschädigende Mittel zur sofortigen kombinierten Verwendung als Medikament zusammen verpackt sind.

14. Kombination nach dem vorhergehenden Anspruch, wobei das gefäßendothelschädigende Mittel ein Combretastatin oder ein Analogon oder Derivat davon ist und das Zelltherapieprodukt mesenchymale Stammzellen (MSZ) und/oder T-Zellen umfasst oder daraus besteht.

## Revendications

1. Produit de thérapie cellulaire comprenant des cellules thérapeutiques de mammifère destiné à être utilisé dans le traitement d'une maladie tumorale solide, d'une maladie associée à une inflammation ou à un trouble du système nerveux central (SNC) ou à un trouble neurologique ou neurodégénératif, dans lequel l'utilisation dudit produit de thérapie cellulaire comprend l'administration d'un agent d'endommagement endothélial vasculaire avant ou en combinaison avec l'administration dudit produit de thérapie cellulaire.

2. Produit de thérapie cellulaire destiné à être utilisé selon la revendication 1, dans lequel l'agent d'endommagement endothélial vasculaire est administré comme adjuvant en une quantité et/ou d'une manière efficace pour améliorer l'enrichissement desdites cellules thérapeutiques à un site cible chez le sujet du traitement, de préférence dans lequel le produit de thérapie cellulaire est administré par voie systémique, de préférence par voie intraveineuse, intra-artérielle et/ou intrapéritonéale.

3. Produit de thérapie cellulaire destiné à être utilisé selon la revendication précédente, dans lequel l'agent d'endommagement endothélial vasculaire est une combrétastatine ou un analogue ou dérivé de celle-ci.

4. Produit de thérapie cellulaire destiné à être utilisé selon la revendication précédente, dans lequel l'analogue ou dérivé de la combrétastatine est le 1-méthyl-5-(3-amino-4-éthoxyphényl)-4-(3-bromo-4,5-diméthoxyphényl)-imidazole bis(chlorhydrate) ou 1-méthyl-5-(3-amino-4-méthoxyphényl)-4-(3-bromo-4,5-diméthoxyphényl)-imidazole bis(chlorhydrate).

5. Produit de thérapie cellulaire destiné à être utilisé selon l'une quelconque des revendications précédentes, dans lequel le produit de thérapie cellulaire est administré pendant 0,5 à 72 heures, de préférence 1 à 24 heures, de manière davantage préférée 2 à 12 heures après l'administration dudit agent d'endommagement endothélial vasculaire.

6. Produit de thérapie cellulaire destiné à être utilisé selon l'une quelconque des revendications précédentes, dans lequel le produit de thérapie cellulaire comprend ou est constitué de cellules souches mésenchymateuses (CSM).

7. Produit de thérapie cellulaire destiné à être utilisé selon la revendication précédente, dans lequel les CSM sont génétiquement modifiées pour exprimer un produit génique thérapeutique à partir d'un acide nucléique exogène.

8. Produit de thérapie cellulaire destiné à être utilisé selon l'une quelconque des revendications 1 à 5, dans lequel le produit de thérapie cellulaire comprend ou est constitué de cellules T.

9. Produit de thérapie cellulaire destiné à être utilisé selon la revendication précédente, dans lequel les cellules T sont des cellules T chimériques de récepteur d'antigène (CAR-T) .

10. Produit de thérapie cellulaire destiné à être utilisé selon l'une quelconque des revendications précédentes, dans lequel un ou plusieurs produits de thérapie cellulaire comprenant des CSM et des CAR-T sont administrés en combinaison.

11. Composition comprenant un produit de thérapie cellulaire comprenant des cellules thérapeutiques de mammifère et un agent d'endommagement endothélial vasculaire à utiliser comme médicament.

12. Composition selon la revendication précédente, dans laquelle l'agent d'endommagement endothélial vasculaire est une combrétastatine ou un analogue ou dérivé de celle-ci et le produit de thérapie cellulaire comprend ou est constitué de cellules souches mésenchymateuses (CSM) et/ou de cellules T.

13. Combinaison d'un produit de thérapie cellulaire comprenant des cellules thérapeutiques de mammifère et un agent d'endommagement endothélial vasculaire, dans laquelle ledit produit de thérapie cellulaire et l'agent d'endommagement endothélial vasculaire sont conditionnés ensemble pour une utilisation combinée immédiate en tant que médicament.

14. Combinaison selon la revendication précédente, dans laquelle l'agent d'endommagement endothélial vasculaire est une combrétastatine ou un analogue ou dérivé de celle-ci et le produit de thérapie cellulaire comprend ou est constitué de cellules souches mésenchymateuses (CSM) et/ou de cellules T.
